# EUROPEAN PATENT SPECIFICATION

(11) **EP 2 134 704 B1**
(45) Date of publication and mention of the grant of the patent: **22.09.2010**
(21) Application number: 08731470.4
(22) Date of filing: 05.03.2008
(51) Int. Cl.: C07D 401/14, C07D 413/14, C07D 417/14, A61K 31/445, A61P 3/10

(54) **COMPOUNDS AND COMPOSITIONS AS MODULATORS OF GPR119 ACTIVITY**
VERBINDUNGEN UND ZUSAMMENSETZUNGEN ALS MODULATOREN DER GPR119-AKTIVITÄT
COMPOSES ET COMPOSITIONS EN TANT QUE MODULATEURS DE L'ACTIVITE DE GPR119

(30) Priority: 08.03.2007 US 893854 P
(43) Date of publication of application: 23.12.2009
(73) Proprietor: IRM LLC, Hamilton HM LX (BM)
(72) Inventor: ALPER, Phillip, B., San Diego, California 92130 (US); LELAIS, Gerald, San Diego, California 92130 (US); EPPLE, Robert, San Diego, California 92122 (US); MUTNICK, Daniel, San Diego, California 92116 (US)
(74) Representative: Kiddle, Simon John
(86) International application number: PCT/US2008/055958
(87) International publication number: WO 2008/109702

(56) References cited:
- WO-A-2007/003960
- WO-A-2007/003961

## Description

### BACKGROUND OF THE INVENTION

### Field of the Invention

The invention provides compounds, pharmaceutical compositions comprising such compounds and such compounds for prevention or treatment of diseases or disorders associated with the activity of GPR119.

### Background

GPR119 is a G-protein coupled receptor (GPCR) that is mainly expressed in the pancreas, small intestine, colon and adipose tissue. The expression profile of the human GPR119 receptor indicates its potential utility as a target for the treatment of obesity and diabetes. The novel compounds of this invention modulate the activity of GPR119 and are, therefore, expected to be useful in the treatment of GPR119-associated diseases or disorders such as, but not limited to, diabetes, obesity and associated metabolic disorders.
WO 2007/003960 describes a range of G-protein coupled receptor (GPCR) agonists which have a 5-membered heteroaryl ring coupled to a phenyl or 5-/6-membered heteroaryl group and also to a carbocyclic or heterocyclic group. WO 2007/003961 also describes a range of GPCR agonists in which a 5-/6-membered heteroaromatic group is attached to a carbocyclic or heterocyclic group and also to a bicyclic group.

### SUMMARY OF THE INVENTION

In one aspect, the present invention provides a compound of Formula **I:**

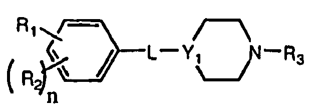

in which:

n is selected from 0, 1, 2 and 3;

L is selected from: - Y₂X₃-; -OX₁X₃-; -OX₁OX₃-; -OX₁C(O)X₂X₃-;-OX₁C(O)OX₂X₃-; and -OC(O)NR₄X₁X₃-; wherein Y₂ is a 5 to 8 member heterocyclic containing 1 to 3 heteroatoms selected from O, N and S; X₁ and X₂ are independently selected from a bond, C₁₋₆alkylene, C₂₋₆alkenylene, C₃₋₈cycloalkyl and C₁₋₅heteroarylene; R₄ is selected from hydrogen and C₁₋₆alkyl; and X₃ is selected from 1,2,4-oxadiazole, 1,3,4-oxadiazole, oxazole, thiazole and tetrazole;

R₁ is selected from C₁₋₄alkyl, halo-substituted-C₁₋₄alkyl, C₆₋₁₀aryl, - X₄S(O)₀₋₂R₅ₐ, -X₄C(O)OR₅ₐ, -X₄OR₅ₐ, - X₄C(O)R₅ₐ, - X₄C(O)NR₅ₐR_{5b}, -X₄NR_{5c}S(O)₀-₂R₅ₐ, - X₄NR_{5c}C(O)OR₅ₐ, -X₄NR_{5c}C(O)R₅ₐ, and - X₄NR_{5c}C(O)NR₅ₐR_{5b}; wherein R₅ₐ and R_{5b} are independently selected from hydrogen, C₁₋₆alkyl, C₁₋₆alkoxy, halo-substituted-C₁₋₆ alkyl, halo-substituted-C₁₋₆alkoxy and C₁₋₁₀heteroaryl, wherein X₄ is selected from a bond, C₁₋₃alkylene and C₃₋₆cycloalkylene; R_{5c} is selected from hydrogen, C₁₋₆alkyl, C₃₋₆ cycloalkyl, C₃₋₈heterocycloalkyl, C₆₋₁₀aryl and C₁₋₁₀heteroaryl; wherein any alkyl, cycloalkyl, aryl or heteroaryl of R_{5c} can be optionally substituted with 1 to 3 radicals independently selected from halo, C₁₋₆alkyl, C₁₋₆alkoxy, halo-substituted-C₁₋₆alkyl and halo-substituted-C₁₋₆alkoxy;

R₂ is selected from halo, cyano, C₁₋₈alkyl, C₁₋₈alkoxy, halo-substituted-C₁₋₈ alkyl, halo-substituted-C₁₋₈alkoxy and nitro;

R₃ is selected from C₁₋₁₀heteroaryl, -C(O)OR₆ₐ, -C(O)R₆ₐ, -S(O)₀₋₂R₆ₐ, - C(O)R₇ and -C(O)X₅NR₆ₐC(O)OR_{6b}; wherein X₅ is selected from a bond and C₁₋₆ alkylene; R₆ₐ and R_{6b} are independently selected from hydrogen and C₁₋₆alkyl; R₇ is selected from C₃₋₈cycloalkyl and C₆₋₁₀aryl; wherein said heteroaryl of R₃ is optionally substituted with 1 to 3 radicals independently selected from halo, C₁₋₆alkyl, C₁₋₆alkoxy, halo-substituted-C₁₋₆alkyl, halo-substituted-C₁₋₆alkoxy, C₃₋₈heterocycloalkyl and C₁₋₁₀ heteroaryl; and

Y₁ is selected from CR₈ and N; wherein R₈ is selected from hydrogen and C₁₋₆alkyl.

In a second aspect, the present invention provides a pharmaceutical composition which contains a compound of Formula I or a N-oxide derivative, individual isomers and mixture of isomers thereof; or a pharmaceutically acceptable salt thereof, in admixture with one or more suitable excipients.

In a third aspect, the present invention provides a compound of Formula I or a N-oxide derivative, individual isomers and mixture of isomers thereof, or a pharmaceutically acceptable salt thereof, for use in the treatment of a disease in an animal in which modulation of GPR119 activity can prevent, inhibit or ameliorate the pathology and/or symptomology of the diseases.

In a fourth aspect, the present invention provides the use of a compound of Formula I in the manufacture of a medicament for treating a disease in an animal in which GPR119 activity contributes to the pathology and/or symptomology of the disease.

### DETAILED DESCRIPTION OF THE INVENTION

### Definitions

"Alkyl" as a group and as a structural element of other groups, for example halo-substituted-alkyl and alkoxy, can be straight-chained, branched or cyclic. C₁₋₆ alkoxy includes methoxy, ethoxy, and the like. Halo-substituted-C₁₋₆alkyl includes a straight chain or branched alkyl chain of between 1 and 6 carbon atoms where some or all of the hydrogens are substituted with a halo group, for example, trifluoromethyl, pentafluoroethyl, and the like. C₁₋₄alkylene, is a divalent straight or branched alkyl chain containing 1 to 4 carbon atoms.

"Aryl" means a monocyclic or fused bicyclic aromatic ring assembly containing six to ten ring carbon atoms. For example, aryl can be phenyl or naphthyl, preferably phenyl. "Arylene" means a divalent radical derived from an aryl group. "Heteroaryl" is as defined for aryl where one or more of the ring members are a heteroatom. For example, C₁₋₁₀heteroaryl includes pyridyl, indolyl, indazolyl, quinoxalinyl, quinolinyl, benzofuranyl, benzopyranyl, benzothiopyranyl, benzo[1,3]dioxole, imidazolyl, benzo-imidazolyl, pyrimidinyl, furanyl, oxazolyl, isoxazolyl, triazolyl, tetrazolyl, pyrazolyl, thienyl, 1H-pyridin-2-onyl, 6-oxo-1,6-dihydro-pyridin-3-yl, etc. "C₆₋₁₀arylC₀₋₄alkyl" means an aryl as described above connected via a alkylene grouping. For example, C₆₋₁₀arylC₀₋₄alkyl includes phenethyl, benzyl, etc. Heteroaryl also includes the N-oxide derivatives, for example, pyridine N-oxide derivatives with the following structure:

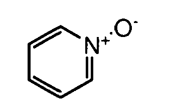

"Cycloalkyl" means a saturated or partially unsaturated, monocyclic, fused bicyclic or bridged polycyclic ring assembly containing the number of ring atoms indicated. For example, C₃₋₁₀cycloalkyl includes cyclopropyl, cyclobutyl, cyclopentyl, cyclohexyl, etc. "Heterocycloalkyl" means cycloalkyl, as defined in this application, provided that one or more of the ring carbons indicated, are replaced by a moiety selected from -O-, -N=, -NR-, -C(O) -, -S-, -S(O) - or -S(O)₂-, wherein R is hydrogen, C₁₋₄alkyl or a nitrogen protecting group. For example, C₃₋₈heterocycloalkyl as used in this application to describe compounds of the invention includes morpholino, pyrrolidinyl, piperazinyl, piperidinyl, piperidinylone, 1,4-dioxa-8-aza-spiro[4.5]dec-8-yl, 2-oxopyrrolidin-1-yl, 2-oxo-piperidin-1-yl, etc.

GPR119 means G protein-coupled receptor 119 (GenBank^{®} Accession No. AAP72125) is also referred to in the literature as RUP3 and GPR116. The term GPR119 as used herein includes the human sequences found in GeneBank accession number AY288416, naturally-occurring allelic variants, mammalian orthologs, and recombinant mutants thereof.

"Halogen" (or halo) preferably represents chloro or fluoro, but can also be bromo or iodo.

"Treat", "treating" and "treatment" refer to a method of alleviating or abating a disease and/or its attendant symptoms.

### Description of the Preferred Embodiments

The present invention provides compounds and, compositions of formula I and such compounds and compositions for use in the treatment of diseases in which modulation of GPR119 activity can prevent, inhibit or ameliorate the pathology and/or symptomology of the diseases.

In one embodiment, with reference to compounds of Formula I, n is selected from 0, 1 and 2;

L is selected from -Y₂X₃- and -OX₁X₃-; wherein Y₂ is a 5 to 8 member heterocyclic containing 1 to 3 heteroatoms selected from O, N and S; X₁ is selected from a bond, C₁₋₆alkylene and C₂₋₆alkenylene; and X₃ is selected from 1,2,4-oxadiazole, 1,3,4-oxadiazole, oxazole, thiazole and tetrazole;

R₁ is selected from halo, cyano, C₁₋₄alkyl, halo-substituted-C₁₋₄alkyl, C₆₋₁₀ aryl, -S(O)₀₋₂R₅ₐ, -C(O)OR₅ₐ, -C(O)R₅ₐ, and -C(O)NR₅ₐR_{5b}; wherein R₅ₐ and R_{5b} are independently selected from hydrogen, C₁₋₄alkyl and halo-substituted-C₁₋₄alkyl;

R₂ is selected from halo and nitro;

R₃ is selected from C₁₋₁₀heteroaryl, -C(O)OR₆ₐ, -C(O)R₆ₐ, -S(O)₀₋₂R₆ₐ, - C(O)R₇ and -C(O)X₅NR₆ₐC(O)OR_{6b}; wherein X₅ is selected from a bond and C₁₋₄ alkylene; R₆ₐ and R_{6b} are independently selected from hydrogen and C₁₋₆alkyl; R₇ is selected from C₃₋₈cycloalkyl and C₆₋₁₀aryl; wherein said heteroaryl of R₃ is optionally substituted with up to three C₁₋₆alkyl radicals; and

Y₁ is selected from CR₈ and N; wherein R₈ is selected from hydrogen and C₁₋₄alkyl.

In another embodiment, L is selected from -Y₂X₃- and -OX₁X₃-; wherein Y₂ is pyrrolidine; X₁ is selected from methylene, propylene, butylene and pentylene; and X₃ is selected from 1,2,4-oxadiazole, 1,3,4-oxadiazole, oxazole, thiazole and tetrazole.

In another embodiment, R₁ is selected from halo, cyano, trifluoromethyl, methyl-sulfonyl, methoxy-carbonyl, phenyl, methyl-carbonyl, amino-carbonyl and trifluoromethyl-sulfonyl.

In another embodiment, R₃ is selected from t-butoxy-carbonyl, isopropoxy-carbonyl, t-butyl-carbonyl, t-butyl-methyl-carbonyl, t-butoxy-carbonylaminomethyl-carbonyl, phenyl-carbonyl, cyclohexyl-carbonyl, t-butyl-sulfinyl, pyridinyl, isopropyl-sulfonyl and ethyl-pyrimidinyl.

In another embodiment are selected from: tert-butyl 4-(3-(1-(4-(methylsulfonyl)phenyl)pyrrolidin-3-yl)-1,2,4-oxadiazol-5-yl)piperidine-1-carboxylate; (S)-isopropyl 4-(3-(1-(2-fluoro-4-(methylsulfonyl)phenyl)pyrrolidin-3-yl)- 1,2,4-oxadiazol-5-yl)piperidine- I -carboxylate; (S)-tert-butyl 4-(5-(1-(2-fluoro-4-(methylsulfonyl)phenyl)pyrrolidin-3-yl)-1,2,4-oxadiazol-3-yl)piperidine- 1-carboxylate; (S)-isopropyl 4-(5-(1-(2-fluoro-4-(methylsulfonyl)phenyl)pyrrolidin-3-yl)-1,2,4-oxadiazol-3-yl)piperidine-1-carboxylate; (S)-3-(1-(5-ethylpyridin-2-yl)piperidin-4-yl)-5-(1-(2-fluoro-4-(methylsulfonyl)phenyl)pyrrolidin-3-yl)-1,2,4-oxadiazole; (S)-1-methylcyclopropyl 4-(5-(1-(2-fluoro-4-(methylsulfonyl)phenyl)pyrrolidin-3-yl)-1,2,4-oxadiazol-3-yl)piperidine-1-carboxylate; (S)-tert-butyl 4-(5-(1-(4-(methylsulfonyl)phenyl)pyrrolidin-3-yl)-1,2,4-oxadiazol-3-yl)piperidine-1-carboxylate; (S)-1-methylcyclopropyl 4-(5-(1-(4-(methylsulfonyl)phenyl)pyrrolidin-3-yl)-1,2,4-oxadiazol-3-yl)piperidine-1-carboxylate; (S)-3-(1-(5-ethylpyrimidin-2-yl)piperidin-4-yl)-5-(1-(4-(methylsulfonyl)phenyl)pyrrolidin-3-yl)-1,2,4-oxadiazole; (S)-isopropyl 4-(5-(1-(2-fluoro-4-(methylsulfonyl)phenyl)pyrrolidin-3-yl)-1,3,4-oxadiazol-2-yl)piperidine-1-carboxylate; (S)-isopropyl 4-(4-(1-(2-fluoro-4-(methylsulfonyl)phenyl)pyrrolidin-3-yl)oxazol-2-yl)piperidine-1-carboxylate; (S)-isopropyl 4-(4-(1-(2-fluoro-4-(methylsulfonyl)phenyl)pyrrolidin-3-yl)thiazol-2-yl)piperidine-1-carboxylate; 4-{2-[1-(4-Methanesulfonyl-phenyl)-pyrrolidin-3-yl]-*S*-oxazol-4-yl}-piperidine-1-carboxylic acid isopropyl ester; 4-{2-[1-(2-Fluoro-4-methanesulfonyl-phenyl)-pyrrolidin-3-yl]-*S*-thiazol-4-yl}-piperidine-1-carboxylic acid isopropyl ester; (S)-isopropyl 4-(2-(1-(2-fluoro-4-(methylsulfonyl)phenyl)pyrrolidin-3-yl)-2H-tetrazol-5-yl)piperidine-1-carboxylate; isopropyl 4-(5-((4-(methylsulfonyl)phenoxy)methyl)-1,2,4-oxadiazol-3-yl)piperidine-1-carboxylate; isopropyl 4-(5-(3-(4-(methylsulfonyl)phenoxy)propyl)-1,2,4-oxadiazol-3-yl)piperidine-1-carboxylate; isopropyl 4-(3-(4-(4-(methylsulfonyl)phenoxy)butyl)-1,2,4-oxadiazol-5-yl)piperidine-1-carboxylate; and isopropyl 4-(3-(5-(4-(methylsulfonyl)phenoxy)pentyl)-1,2,4-oxadiazol-5-yl)piperidine-1-carboxylate.

Further compounds of Formula I are detailed in the Examples and Tables, *infra.*

### Pharmacology and Utility

Compounds of the invention modulate the activity of GPR119 and, as such, are useful for treating diseases or disorders in which the activity of GPR119 contributes to the pathology and/or symptomology of the disease. This invention further provides compounds of this invention for use in the preparation of medicaments for the treatment of diseases or disorders in which GPR119 activity contributes to the pathology and/or symptomology of the disease.

The resultant pathologies of Type 11 diabetes are impaired insulin signaling at its target tissues and failure of the insulin-producing cells of the pancreas to secrete an appropriate degree of insulin in response to a hyperglycemic signal. Current therapies to treat the latter include inhibitors of the β-cell ATP-sensitive potassium channel to trigger the release of endogenous insulin stores, or administration of exogenous insulin. Neither of these achieves accurate normalization of blood glucose levels and both carry the risk of inducing hypoglycemia. For these reasons, there has been intense interest in the development of pharmaceuticals that function in a glucose-dependent action, i.e. potentiators of glucose signaling. Physiological signaling systems which function in this manner are well-characterized and include the gut peptides GLP-1, GIP and PACAP. These hormones act via their cognate G-protein coupled receptor to stimulate the production of cAMP in pancreatic β-cells. The increased cAMP does not appear to result in stimulation of insulin release during the fasting or pre-prandial state. However, a series of biochemical targets of cAMP signaling, including the ATP-sensitive potassium channel, voltage-sensitive potassium channels and the exocytotic machinery, are modified in such a way that the insulin secretory response to a postprandial glucose stimulus is markedly enhanced. Accordingly, agonists of novel, similarly functioning, β-cell GPCRs, including GPR119, would also stimulate the release of endogenous insulin and consequently promote normoglycemia in Type II diabetes. It is also established that increased cAMP, for example as a result of GLP-1 stimulation, promotes β-cell proliferation, inhibits β-cell death and thus improves islet mass. This positive effect on β-cell mass is expected to be beneficial in both Type II diabetes, where insufficient insulin is produced, and Type I diabetes, where β-cells are destroyed by an inappropriate autoimmune response.

Some β-cell GPCRs, including GPR119, are also present in the hypothalamus where they modulate hunger, satiety, decrease food intake, controlling or decreasing weight and energy expenditure. Hence, given their function within the hypothalamic circuitry, agonists or inverse agonists of these receptors mitigate hunger, promote satiety and therefore modulate weight.

It is also well-established that metabolic diseases exert a negative influence on other physiological systems. Thus, there is often the codevelopment of multiple disease states (e.g. type I diabetes, type II diabetes, inadequate glucose tolerance, insulin resistance, hyperglycemia, hyperlipidemia, hypertriglyceridemia, hypercholesterolemia, dyslipidemia, obesity or cardiovascular disease in "Syndrome X") or secondary diseases which clearly occur secondary to diabetes (e.g. kidney disease, peripheral neuropathy). Thus, it is expected that effective treatment of the diabetic condition will in turn be of benefit to such interconnected disease states.

In an embodiment of the invention is a compound of the invention or a pharmaceutical composition thereof for use in the treatment of a metabolic disease and/or a metabolic-related disorder in an individual. The metabolic diseases and metabolic-related disorders are selected from, but not limited to, hyperlipidemia, type 1 diabetes, type 2 diabetes mellitus, idiopathic type 1 diabetes (Type Ib), latent autoimmune diabetes in adults (LADA), early-onset type 2 diabetes (EOD), youth-onset atypical diabetes (YOAD), maturity onset diabetes of the young (MODY), malnutrition-related diabetes, gestational diabetes, coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis, ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertrygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance.

In accordance with the foregoing, the present invention further provides a therapeutically effective amount (*See, "Administration and Pharmaceutical Compositions", infra)* of a compound of Formula **I** or a pharmaceutically acceptable salt thereof for use in preventing or ameliorating the symptamology of any of the diseases or disorders described above in a subject in need thereof. For any of the above uses, the required dosage will vary depending on the mode of administration, the particular condition to be treated and the effect desired.

### Administration and Pharmaceutical Compositions

In general, compounds of the invention will be administered in therapeutically effective amounts via any of the usual and acceptable modes known in the art, either singly or in combination with one or more therapeutic agents. A therapeutically effective amount can vary widely depending on the severity of the disease, the age and relative health of the subject, the potency of the compound used and other factors. In general, satisfactory results are indicated to be obtained systemically at daily dosages of from about 0.03 to 2.5mg/kg per body weight. An indicated daily dosage in the larger mammal, e.g. humans, is in the range from about 0.5mg to about 100mg, conveniently administered, e.g. in divided doses up to four times a day or in retard form. Suitable unit dosage forms for oral administration comprise from ca. 1 to 50mg active ingredient.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrollidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions can be sterilized and/or contain adjuvants, such as preserving, stabilizing, wetting or emulsifying agents, solution promoters, salts for regulating the osmotic pressure and/or buffers. In addition, they can also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations can also be used. Suitable formulations for topical application, e.g., to the skin and eyes, are preferably aqueous solutions, ointments, creams or gels well-known in the art. Such can contain solubilizers, stabilizers, tonicity enhancing agents, buffers and preservatives.

Compounds of the invention can be administered in therapeutically effective amounts in combination with one or more therapeutic agents (pharmaceutical combinations).

For example, synergistic effects can occur with other anti-obesity agents, anorectic agents, appetite suppressant and related agents. Diet and/or exercise can also have synergistic effects. Anti-obesity agents include, but are not limited to, apolipoprotein-B secretion/microsomal triglyceride transfer protein (apo-B/MTP) inhibitors, MCR-4 agonists, cholescystokinin-A (CCK-A) agonists, serotonin and norepinephrine reuptake inhibitors (for example, sibutramine), sympathomimetic agents, β3 adrenergic receptor agonists, dopamine agonists (for example, bromocriptine), melanocyte-stimulating hormone receptor analogs, cannabinoid 1 receptor antagonists [for example, compounds described in WO2006/047516), melanin concentrating hormone antagonists, leptons (the OB protein), leptin analogues, leptin receptor agonists, galanin antagonists, lipase inhibitors (such as tetrahydrolipstatin, i.e., Orlistat), anorectic agents (such as a bombesin agonist), Neuropeptide-Y antagonists, thyromimetic agents, dehydroepiandrosterone or an analogue thereof, glucocorticoid receptor agonists or antagonists, orexin receptor antagonists, urocortin binding protein antagonists, glucagon-like peptide- 1 receptor agonists, ciliary neutrotrophic factors (such as Axokine^{™}), human agouti-related proteins (AGRP), ghrelin receptor antagonists, histamine 3 receptor antagonists or reverse agonists, neuromedin U receptor agonists, noradrenergic anorectic agents (for example, phentermine, mazindol and the like) and appetite suppressants (for example, bupropion).

Where compounds of the invention are administered in conjunction with other therapies, dosages of the co-administered compounds will of course vary depending on the type of co-drug employed, on the specific drug employed, on the condition being treated and so forth.

A combined preparation or pharmaceutical composition can comprise a compound of the invention as defined above or a pharmaceutical acceptable salt thereof and at least one active ingredient selected from:

a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; DPPIV (dipeptidyl peptidase IV) inhibitors such as DPP728, LAF237 (vildagliptin - Example 1 of WO 00/34241), MK-0431, saxagliptin, GSK23A ; an AGE breaker; a thiazolidone derivative (glitazone) such as pioglitazone, rosiglitazone, or (R)-1-{4-[5-methyl-2-(4-trifluoromethyl-phenyl)-oxazol-4-ylmethoxy]-benzenesulfonyl}-2,3-dihydro-1*H*-indole-2-carboxylic acid described in the patent application WO 03/043985, as compound 19 of Example 4, a non-glitazone type PPAR gamma agonist e.g. GI-262570; Diacylglycerol acetyltransferase (DGAT) inhibitors such as those disclosed in WO 2005044250, WO 2005013907, WO 2004094618 and WO 2004047755;

b) hypolipidemic agents such as 3-hydroxy-3-methyl-glutaryl coenzyme A (HMG-CoA) reductase inhibitors, e.g., lovastatin and related compounds such as those disclosed in U.S. Pat. No. 4,231,938, pitavastatin, simvastatin and related compounds such as those disclosed in U.S. Pat. Nos. 4,448,784 and 4,450,171, pravastatin and related compounds such as those disclosed in U.S. Pat. No.4,346,227, cerivastatin, mevastatin and related compounds such as those disclosed in U.S. Pat. No. 3,983,140, velostatin, fluvastatin, dalvastatin, atorvastatin, rosuvastatin and related statin compounds disclosed in U.S. Pat. No. 5,753,675, rivastatin, pyrazole analogs of mevalonolactone derivatives as disclosed in U.S. Pat. No. 4,613,610, indene analogs of mevalonolactone derivatives as disclosed in PCT application WO 86/03488, 6-[2-(substituted-pyrrol-1-yl)-alkyl)pyran-2-ones and derivatives thereof as disclosed in U.S. Pat. No. 4,647,576, Searle's SC-45355 (a 3- substituted pentanedioic acid derivative) dichloroacetate, imidazole analogs of mevalonolactone as disclosed in PCT application WO 86/07054, 3- carboxy-2- hydroxypropane-phosphonic acid derivatives as disclosed in French Patent No. 2,596,393, 2,3-disubstituted pyrrole, furan and thiophene derivatives as disclosed in European Patent Application No. 0221025, naphthyl analogs of mevalonolactone as disclosed in U.S. Pat. No. 4,686,237, octahydronaphthalenes such as disclosed in U.S. Pat. No. 4, 499,289, keto analogs of mevinolin (lovastatin) as disclosed in European Patent Application No.0,142,146 A2, and quinoline and pyridine derivatives disclosed in U.S. Pat. Nos. 5,506,219 and 5,691,322. In addition, phosphinic acid compounds useful in inhibiting HMG CoA reductase suitable for use herein are disclosed in GB 2205837; squalene synthase inhibitors; FXR (farnesoid X receptor) and LXR (liver X receptor) ligands; cholestyramine; fibrates; nicotinic acid and aspirin;

c) an anti-obesity agent or appetite regulating agent such as a CB1 activity modulator, melanocortin receptor (MC4R) agonists, melanin-concentrating hormone receptor (MCHR) antagonists, growth hormone secretagogue receptor (GHSR) antagonists, galanin receptor modulators, orexin antagonists, CCK agonists, GLP-1 agonists, and other Pre-proglucagon-derived peptides; NPY1 or NPY5 antagonsist, NPY2 and NPY4 modulators, corticotropin releasing factor agonists, histamine receptor-3 (H3) modulators, aP2 inhibitors, PPAR gamma modulators, PPAR delta modulators, acetyl-CoA carboxylase (ACC) inihibitors, 11-β-HSD-1 inhibitors, adinopectin receptor modulators; beta 3 adrenergic agonists, such as AJ9677 (Takeda/Dainippon), L750355 (Merck), or CP331648 (Pfizer) or other known beta 3 agonists as disclosed in U.S. Pat. Nos. 5,541,204, 5,770,615, 5, 491,134, 5,776,983 and 5,488,064, a thyroid receptor beta modulator, such as a thyroid receptor ligand as disclosed in WO 97/21993 (U. Cal SF), WO 99/00353 (KaroBio) and GB98/284425 (KaroBio), a SCD-1 inhibitor as disclosed in WO2005011655, a lipase inhibitor, such as orlistat or ATL-962 (Alizyme), serotonin receptor agonists, (e.g., BVT- 933 (Biovitrum)), monoamine reuptake inhibitors or releasing agents, such as fenfluramine, dexfenfluramine, fluvoxamine, fluoxetine, paroxetine, sertraline, chlorphentermine, cloforex, clortermine, picilorex, sibutramine, dexamphetamine, phentermine, phenylpropanolamine or mazindol, anorectic agents such as topiramate (Johnson & Johnson), CNTF (ciliary neurotrophic factor)/Axokine® (Regeneron), BDNF (brain-derived neurotrophic factor), leptin and leptin receptor modulators, phentermine, leptin, bromocriptine, dexamphetamine, amphetamine, fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine;

d) anti-hypertensive agents such as loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168; beta-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; aldosterone synthase inhibitors; and dual ET/All antagonist such as those disclosed in WO 00/01389.

e) a HDL increasing compound;

f) Cholesterol absorption modulator such as Zetia® and KT6-971;

g) Apo-A1 analogues and mimetics;

h) thrombin inhibitors such as Ximelagatran;

i) aldosterone inhibitors such as anastrazole, fadrazole, eplerenone;

j) Inhibitors of platelet aggregation such as aspirin, clopidogrel bisulfate;

k) estrogen, testosterone, a selective estrogen receptor modulator, a selective androgen receptor modulator;

1) a chemotherapeutic agent such as aromatase inhibitors e.g. femara, antiestrogens, topoisomerase I inhibitors, topoisomerase II inhibitors, microtubule active agents, alkylating agents, antineoplastic anti metabolites, platin compounds, compounds decreasing the protein kinase activity such as a PDGF receptor tyrosine kinase inhibitor preferably Imatinib ({N-{5-[4-(4-methyl-piperazino-methyl)-benzoylamido]-2-methylphenyl}-4-(3-pyridyl)-2-pyrimidine-amine}) described in the European patent application EP-A-0 564 409 as example 21 or 4-Methyl-N-[3-(4-methyl-imidazol-1-yl)-5-trifluoromethyl-phenyl]-3-(4-pyridin-3-yl-pyrimidin-2-ylamino)-benzamide described in the patent application WO 04/005281 as example 92; and

m) an agent interacting with a 5-HT₃ receptor and/or an agent interacting with 5-HT₄ receptor such as tegaserod described in the US patent No. 5510353 as example 13, tegaserod hydrogen maleate, cisapride, cilansetron;

n) an agent for treating tobacco abuse, e.g., nicotine receptor partial agonists, bupropion hypochloride (also known under the tradename Zyban®) and nicotine replacement therapies;

o) an agent for treating erectile dysfunction, e.g., dopaminergic agents, such as apomorphine), ADD/ADHD agents (e.g., Ritalin®, Strattera®, Concerta® and Adderall®);

p) an agent for treating alcoholism, such as opioid antagonists (e.g., naltrexone (also known under the tradename ReVia®) and nalmefene), disulfiram (also known under the tradename Antabuse®), and acamprosate (also known under the tradename Campral®)). In addition, agents for reducing alcohol withdrawal symptoms may also be co-administered, such as benzodiazepines, beta- blockers, clonidine, carbamazepine, pregabalin, and gabapentin (Neurontin®);

q) other agents that are useful including anti-inflammatory agents (e.g., COX-2 inhibitors) ; antidepressants (e.g., fluoxetine hydrochloride (Prozac®)); cognitive improvement agents (e.g., donepezil hydrochloride (Aircept®) and other acetylcholinesterase inhibitors); neuroprotective agents (e.g., memantine) ; antipsychotic medications (e.g., ziprasidone (Geodon®), risperidone (Risperdal®), and olanzapine (Zyprexa®));

or, in each case a pharmaceutically acceptable salt thereof; and optionally a pharmaceutically acceptable carrier.

The invention also provides for a pharmaceutical combinations, e.g. a kit, comprising a) a first agent which is a compound of the invention as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g. a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Processes for Making Compounds of the Invention

The present description also provides processes for the preparation of compounds of the invention. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

In the following schemes, several methods of preparing the compounds of the present invention are illustrative. One of skill in the art will appreciate that these methods are representative, and in no way inclusive of all methods for preparing the compounds of the present invention. The radicals in the schemes are as described in Formula I. The starting compounds for the schemes can all be prepared by standard reactions which are familiar to those in the art or are commercially available.

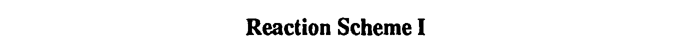

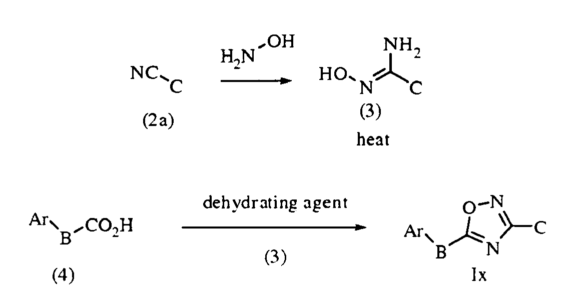

A compound of Formula Ix can be prepared as in reaction scheme I by reacting a compound of formula 2a with hydroxylamine in a suitable solvent such as ethanol and the like at an elevated temperature such as 80 °C to generate a hydroxyamidine of the formula 3. Then, a compound of the formula 4 (where Ar could stand for a protecting group on a heteroatom that will later be removed and functionalized with the appropriate aryl group) can be treated with a dehydrating agent such as carbonyl diimidazole (CDI) followed by stirring for up to 3 hours followed by treatment with a compound of the formula 3 and stirring for up to 12 hours followed by additional CDI and stirring at elevated temperature (around 110°C) in a suitable solvent such as dioxane, DMF and the like. Alternatively, a compound of Formula Ix can be prepared by reacting a compound of the formula 4 with a compound of formula 3 in the presence of a carbodiimide in suitable solvent (for example, DMF, and the like) followed by heating (around 110 °C) in a similar solvent with or without isolation of the intermediate for a period of up to 12 hours. Additionally, an isolable active ester of a compound of the formula 4 may be formed by reacting this compound with a dehydrating agent such as a carbodiimide or the like with a suitable leaving group such as N-hydroxysuccinimide or the like in a suitable solvent and then the isolated active ester may be heated with a compound of the formula 3 in a suitable solvent such as DMF or dioxane at a temperature from 60°C to 120°C for a period of up to 12 hours. In addition to the described methods, any of the synthetic methods known in the art of oxadiazole synthesis (such as Science of Synthesis 2004, 13, 127, Tetrahedron Lett. 2006, 47, 3629 and J. Med. Chem. 2004, 47, 5821) may be employed.

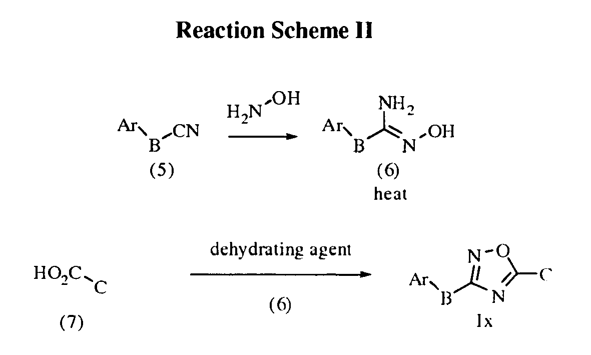

A compound of the formula Ix may be prepared as in reaction scheme II by reversing the order of the nitrile and carboxylic acid in reaction scheme I. A compound of the formula 5 (where Ar could stand for a protecting group on a heteroatom that will later be removed and functionalized with the appropriate aryl group) can be reacted with hydroxylamine in a suitable solvent such as ethanol and the like at an elevated temperature such as 80 °C to afford a compound of the formula 6. Then, the desired compound Ix can be formed by reacting a compound of the formula 7 with a compound of the formula 6 by using any of the methods mentioned in reaction scheme I.

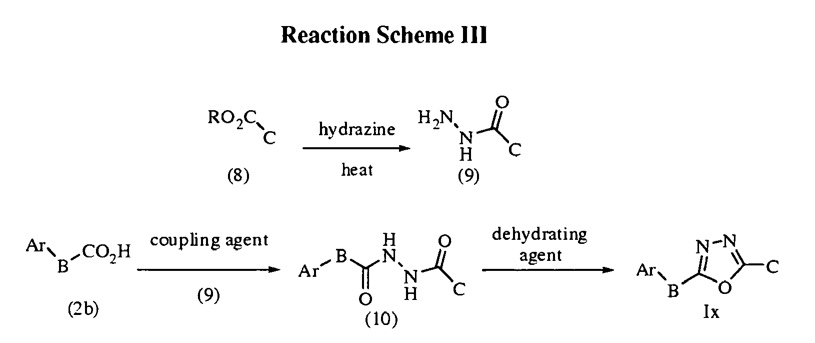

A compound of the formula Ix may be prepared as in reaction scheme III by reacting a compound of the formula 8 with hydrazine in a suitable solvent such as ethanol and the like at elevated temperature to generate a compound of the formula 9. This intermediate can then be reacted with a compound of the formula 2b (where Ar could stand for a protecting group on a heteroatom that will later be removed and functionalized with the appropriate aryl group) along with a coupling agents such as a carbodiimide or the like in an appropriate solvent such as DCM, DMF and the like to generate an intermediate of the formula 10 (where Ar could stand for a protecting group on a heteroatom that will later be removed and functionalized with the appropriate aryl group). This intermediate can then be dehydrated by heating with a dehydrating agent such as TsCl and the like in an appropriate solvent such as pyridine and the like at an elevated temperature such as 90 °C to afford the desired product.

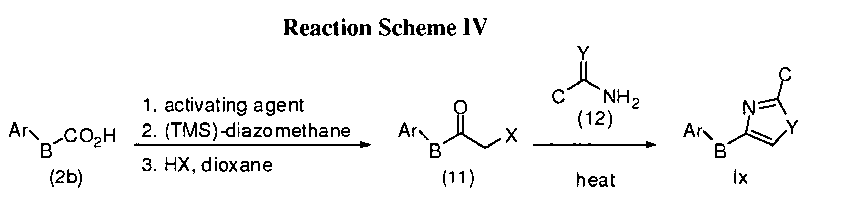

A compound of the formula Ix (where Y can be O or S) can be prepared as in reaction sheme IV by reacting a compound of the formula 2b (where Ar could stand for a protecting group on a heteroatom that will later be removed and functionalized with the appropriate aryl group) with an activating agent such as oxalyl chloride and catalytic DMF in an appropriate solvent such as toluene and the like or isobutyl chloroformate with an appropriate base such as N-methyl morpholine and the like in an appropriate solvent such as THF and the like. The resulting activated acid can then be treated with a solution of TMS-diazomethane or diazomethane in an appropriate solvent such as diethyl ether and the like followed by stirring for up to 12 hours and quenching with a solution of a mineral acid such as HCl or HBr as a solution in an appropriate solvent such as dioxane to generate a haloketone of the formula 11. This haloketone can then be reacted with a compound of the formula 12 where Y can be either O or S either neat or in a solvent such as ethanol, DMF and the like at elevated temperature ranging from 60 °C to 250 °C to afford the desired compounds.

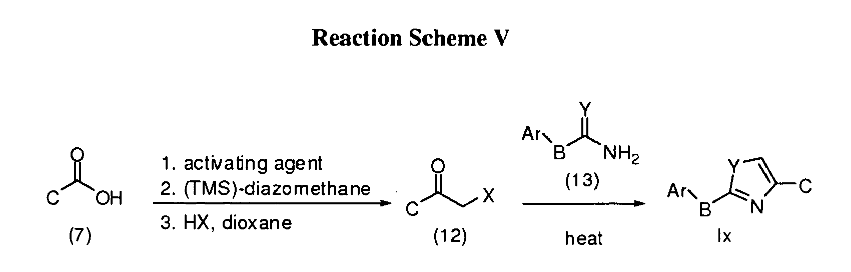

A compound of the formula Ix can be prepared as in reaction scheme V by following the same sequence of steps as in reaction scheme IV except that the haloketone intermediate of the formula 12 is made from a compound of the formula 7 and is reacted with a compound of the formula 13 to generate the desired material.

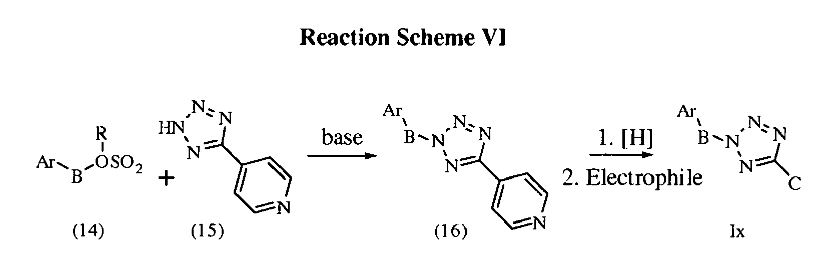

A compound of the formula Ix can be prepared as in reaction scheme VI by reacting an alkyl or arylsulfonyl ester of the formula 14 with the compound of the formula 15 in the presence of a suitable base such as Cs₂CO₃ , K₂CO₃ or the like in a suitable solvent such as NMP and the like at an elevated temperature between 60 and 200 °C for a period of time between 1 minute and 24 hours to generate a compound of the formula 16. The intermediate of the formula 16 can then be reduced to the piperidine by any of the methods known in the literature such as hydrogentation over PtO₂ in AcOH in the presence of a stoichiometric amount of a strong acid such as TFA and the like followed by introduction of an appropriate electophile to generate Ix.

Detailed descriptions of the synthesis of compounds of the Invention are given in the Examples, *infra.*

### Additional Processes for Making Compounds of the Invention

A compound of the invention can be prepared as a pharmaceutically acceptable acid addition salt by reacting the free base form of the compound with a pharmaceutically acceptable inorganic or organic acid. Alternatively, a pharmaceutically acceptable base addition salt of a compound of the invention can be prepared by reacting the free acid form of the compound with a pharmaceutically acceptable inorganic or organic base. Alternatively, the salt forms of the compounds of the invention can be prepared using salts of the starting materials or intermediates.

The free acid or free base forms of the compounds of the invention can be prepared from the corresponding base addition salt or acid addition salt form, respectively. For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, or the like) in a suitable inert organic solvent (e.g. acetonitrile, ethanol, aqueous dioxane, or the like) at 0 to 80 °C.

Prodrug derivatives of the compounds of the invention can be prepared by methods known to those of ordinary skill in the art (e.g., for further details see Saulnier et al., (1994), Bioorganic and Medicinal Chemistry Letters, Vol. 4, p. 1985). For example, appropriate prodrugs can be prepared by reacting a non-derivatized compound of the invention with a suitable carbamylating agent (e.g., 1,1-acyloxyalkylcarbanochloridate, para-nitrophenyl carbonate, or the like).

Protected derivatives of the compounds of the invention can be made by means known to those of ordinary skill in the art. A detailed description of techniques applicable to the creation of protecting groups and their removal can be found in T. W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:
(a) that of reaction schemes I-VI; and
(b) optionally converting a compound of the invention into a pharmaceutically acceptable salt;
(c) optionally converting a salt form of a compound of the invention to a non-salt form;
(d) optionally converting an unoxidized form of a compound of the invention into a pharmaceutically acceptable N-oxide;
(e) optionally converting an N-oxide form of a compound of the invention to its unoxidized form;
(f) optionally resolving an individual isomer of a compound of the invention from a mixture of isomers;
(g) optionally converting a non-derivatized compound of the invention into a pharmaceutically acceptable prodrug derivative; and
(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### Examples

The present invention is further exemplified, but not limited, by the following Examples that illustrate the preparation of compounds of the invention.

### Example 1

### tert-butyl 4-(3-(1-(4-(methylsulfonyl)phenyl)pyrrolidin-3-yl)-1,2,4-oxadiazol-5 - yl)piperidine-1-carboxylate

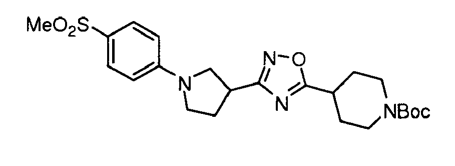

Intermediate 1a: 1-(4-(methylsulfonyl)phenyl)pyrrolidine-3-carbonitrile

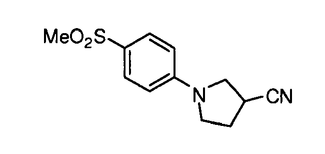

A solution of tert-butyl 3-cyanopyrrolidine-1-carboxylate (449 mg. 2.3 mmol) in dichloromethane (1 mL) is treated with TFA (1 mL) and stirred for 1 hour. The volatiles are then removed by rotary evaporation and the residue is treated with 1-fluoro-4-(methylsulfonyl)benzene (332 mg, 1.9 mmol), K₂CO₃ (790 mg. 5.7 mmol) and DMF (5 mL). The reaction is then heated to 100°C overnight. The reaction is diluted with ethyl acetate and extracted with 1M HCl and water, dried over MSO₄ and the solvent is removed. The material is used crude for the next reaction; ESIMS *m*/*z* for (M-Boc+H)⁺C₁₈H₂₅N₄O₃S calcd.: 197.1 found: 197.1.

Intermediate 1b: N'-hydroxy-1-(4-(methylsulfonyl)phenyl)pyrrolidine-3-carboximidamide

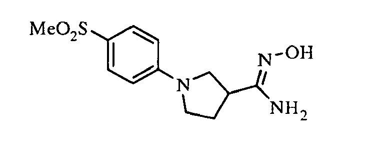

A solution of 1-(4-(methylsulfonyl)phenyl)pyrrolidine-3-carbonitrile (90.3 mg, 0.36 mmol) in ethaonol (2 mL) is treated with aqueous hydroxylamine (23 µL, 0.38 mmol) and heated to 80 °C in ethanol for 3 hours. The reaction is cooled to room temperature and the solvent is removed. The residue is lyophilized and used crude for the next step.

A solution of 1-(tert-butoxycarbonyl)piperidine-4-carboxylic acid (67.9 mg, 0.30 mmol) in DMF (0.5 mL) is treated with a solution of carbonyl diimidazole (52.8 mg, 0.33 mmol) in DMF (0.5 mL) and stirred for 30 minutes. Then, a solution of N'-hydroxy-1-(4-(methylsulfonyl)phenyl)pyrrolidine-3-carboximidamide (92.3 mg, 0.33 mmol) in DMF is added and the reaction is stirred overnight. The reaction is then treated with a solution of carbonyl diimidazole (52.8 mg, 0.33 mmol) in DMF (0.5 mL) and heated to 115 °C for 6 hours. After cooling to room temperature, the reaction is purified by mass triggered preperative HPLC to afford 39.6 mg of the title material. ¹H NMR (400 MHz, CDCl₃) δ 7.75 (d, *J* = 8.9 Hz, 2H), 6.60 (d, *J* = 9.0 Hz, 2H), 4.10 (m, 1H), 3.68 (m, 5H), 3.09 (m, 1H), 3.00 (s, 3H), 2.96 (m, 2H), 2.47 (m, 2H), 2.07 (m, 2H), 1.82 (m, 2H), 1.47 (s, 9H); ESIMS *m*/*z* for (M-Boc+H)⁺C₁₈H₂₅N₄O₃S calcd.: 377.5 found: 377.2

### Example 2

### (S)-isopropyl 4-(3-(1-(2-fluoro-4-(methylsulfonyl)phenyl)pyrrolidin-3-yl)-1,2,4-oxadiazol-5-yl)piperidine-1-carboxylate

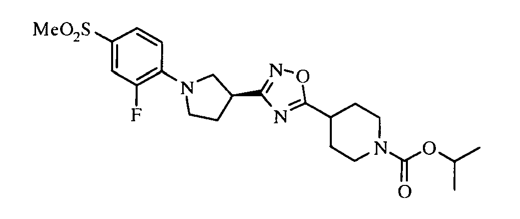

Intermediate 2a: (R)-1-(2-fluoro-4-(methylsulfonyl)phenyl)pyrrolidin-3-ol

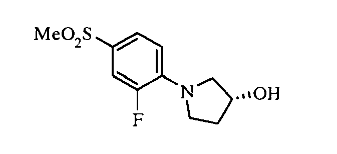

A mixture of 1,2-difluoro methyl sulfonyl benzene (2.484 g, 12.92 mmol), 3-(R)-hydroxypyrrolidine hydrochloride (1.917 g, 15.51 mmol), and K₂CO₃ (4.47 g, 32.31 mmol) in DMF (13 mL) is heated to 100°C and maintained overnight. The reaction is poured over H₂O and extracted with EtOAc. The organics were pooled, dried (MgSO₄), filtered and concentrated. The crude material was purified via SiO₂ chromatography (ISCO, 0-80%, EtOAc in Hexanes) to afford the title compound. ³H NMR (400 MHz, CDCl₃) δ 7.49 (m, 2H), 6.63 (dd, J = 8.6, 8.5 Hz, 1H), 4.59 (m, 1H), 3.73 (m, 2H), 3.52 (m, 2H), 3.00 (s, 3H), 2.08 (m, 2H); LC/MS calcd. for [M+H]⁺C₁₁H₁₅FNO₃S: 260.3, found: 260.0.

Intermediate 2b: (R)-1-(2-fluoro-4-(methylsulfonyl)phenyl)pyrrolidin-3-yl methanesulfonate

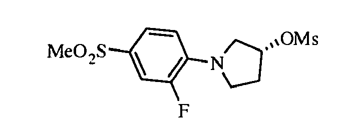

A sample of intermediate 2a (1 g, 3.9 mmol) is treated with dichloromethane (30 mL) and pyridine (915 mg, 11.6 mmol). The reaction is then treated with methanesulfonyl chloride (663 mg, 5.8 mmol) and stirred overnight. The reaction is diluted with ethyl acetate and extracted with 1M HCl twice. The organics are dried over MgSO₄. filtered, evaporated and purified over silica gel using a linear gradient of 0 to 100% ethyl acetate in hexane to afford the title material; ESIMS *m*/*z* for (M⁺+ H⁺) C₁₂H₁₆FNO₅S₂ calcld. 338.1, found 338.2.

Intermediate 2c: (S)-1-(2-fluoro-4-(methylsulfonyl)phenyl)pyrrolidine-3-carbonitrile

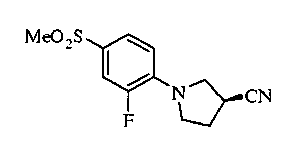

A sample of intermediate 2b (11 g, 33 mmol) is treated with DMF (25 mL), tetrabutylammonium cyanide (1.75 g, 6.5 mmol) and sodium cyanide (1.60 g, 32 mmol). The reaction is heated to 80 °C overnight. An additional portion of tetrabutylammonium cyanide (2 g, 7.5 mmol) is added and heating is continuted for 2 hours. Another 2 g portion of tetrabutylammonium cyanide is added and the reaction is heated another 2 hours. The reaction is cooled to room temperature and diluted with water and ethyl acetate. The aqueous layer is isolated, extracted with 2 more portions of ethyl acetate and discarded. The combined organics are extracted 3 times with 1 M NaOH, dried over MgSO₄, filtered and evaporated. The residue is crystallized from a mixture of ethyl acetate and toluene to afford the nitrile. The mother liquors from the crystallization are used to make intermediate 3a: LC/MS calcd. for [M+H]⁺C₁₂H₁₃FN₂O₂S: 269.1, found: 269.1.

By following the same procedures as example 1 except using intermediate 2c as the nitrile and piperidine-1,4-dicarboxylic acid monoisopropyl ester as the acid equivalent, the desired oxadiazole adduct is obtained. ¹H NMR (400 MHz, CDCl₃) δ 7.52 (m, 2H), 6.67 (dd, *J* = 8.5, 8.4 Hz, 1H), 4.93 (sept., *J* = 6.2 Hz, 1H), 4.14 (m, 2H), 3.95 (m, 1H), 3.84 (m, 1H), 3.73-3.65 (m, 3H), 3.11 (m, 1H), 3.02 (m, 1H), 3.01 (s, 3H), 2.41 (m, 2H), 2.08 (m, 2H), 1.83 (m, 2H), 1.25 (d, *J* = 6.3 Hz, 6H): ESIMS *m*/*z* for (M+H)⁺ C₂₂H₃₀FN₄O₅S calcd.: 481.6 found: 481.2.

### Example 3

### (S)-tert-butyl 4-(5-(1-(2-fluoro-4-(methylsulfonyl)phenyl)pyrrolidin-3-yl)-1,2,4-oxadiazol-3-yl)piperidine- 1-carboxylate

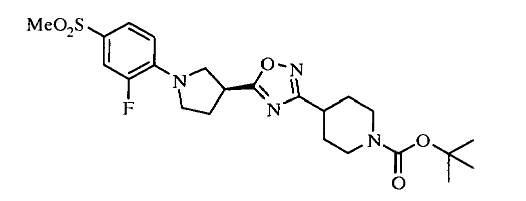

Intermediate 3a: (S)-1-(2-fluoro-4-(methylsulfonyl)phenyl)pyrrolidine-3-carboxylic acid

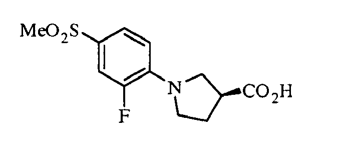

The mother liquor from the crystallization of intermediate 2b is evaporated to dryness and treated with concentrated hydrochloric acid (20 mL). The reaction is then heated to reflux for 1 hour, cooled to room temperature, diluted with water and extracted with ethyl acetate twice. The aqueous layer is discarded and the organics are extracted with 1 M NaOH twice and discarded. The basic aqueous extracts are made acidic with concentrated HCl, extracted with ethyl acetate twice and discarded. The final organics are dried over MgSO₄, evaporated and crystallized from a hot mixture of ethyl acetate and toluene to afford the title material; LC/MS calcd. for [M+H]⁺C₁₂H₁₄FNO₄S: 288.1, found: 288.1.

Intermediate 3b: (S)-2,5-dioxopyrrolidin-1-yl 1-(2-fluoro-4-(methylsulfonyl)phenyl)pyrrolidine-3-carboxylate

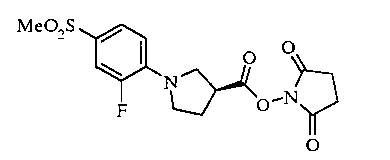

A solution of intermediate 3a (300 mg, 1.2 mmol) and N-hydroxysuccinimide (186 mg, 1.6 mmol) in DMF (3 mL) is treated with EDCI (288 mg, 1.5 mmol) and stirred at room temperature overnight. The reaction is then diluted with ethyl acetate and water. The organics are isolated and extracted with water brine, dried over MgSO₄ and evaporated. The material is used crude for the next transformation. LC/MS calcd. for [M+H]⁺C₁₆H₁₇FN₂O₆S: 384.1, found: 384.1.

A solution of intermediate 3b from the previous step and tert-butyl 4-(N'-hydroxycarbamimidoyl)piperidine-1-carboxylate (281 mg, 1.2 mmol) in dioxane (2.5 mL) is sealed and heated to 85 °C for 4 hours. The solvent is then removed and the residue is purified on silica gel using a linear gradient of 0 to 100 % ethyl acetate in hexane to afford the title material; ¹H NMR (400 MHz, CDCl₃) δ 7.55 (m, 2H), 6.67 (dd, *J* = 8.5, 8.5 Hz, 1H), 4.12 (m, 2H), 3.95 (m, 1H), 4.01 (m, 1H), 3.92 (m,1), 3.74 (m, 3H), 3.02 (s, 3H), 2.91 (m, 3H), 2.48 (m, 2H), 1.98 (m, 2H), 1.47 (s, 9H); ESIMS *m*/*z* for (M+H)⁺C₂₃H₃₁FN₄O₅S calcd.: 495.2 found: 495.2.

### Example 4

### (S)-isopropyl 4-(5-(1-(2-fluoro-4-(methylsulfonyl)phenl)pyrrolidin-3-yl)-1,2,4-oxadiazol-3-yl)piperidine-1-carboxylate

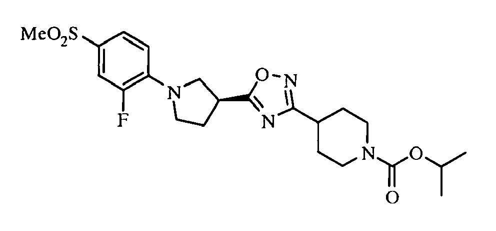

Intermediate 4a: (S)-5-(1-(2-fluoro-4-(methylsulfonyl)phenyl)pyrrolidin-3-yl)-3-(piperidin-4-yl)-1,2,4-oxadiazole

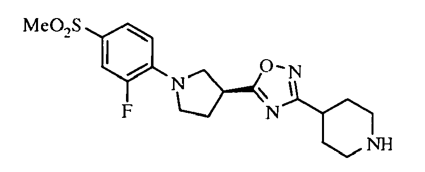

A sample of example 3 (210 mg, 0.43 mmol) is treated with a 4 M solution of HCl in dioxane (3 mL) and the reaction is aged for 2 hours. The solvent is removed and the residue is partitioned between ethyl acetate and 1 M NaOH. The organics are isolated, dried over MgSO₄ and evaporated. The material was used as is. ESIMS *m*/*z* for (M+H)⁺ C₁₈H₂₃FN₄O₃S calcd.: 395.2 found: 395.2.

A solution of intermediate 4a (168 mg, 0.43 mmol) in DCM (4mL) is cooled in an ice/water bath and treated with triethylamine (85 mg, 0.85 mmol) followed by a 1M solution of isopropyl chloroformate in toluene (426 µL, 0.43 mmol). The reaction is allowed to warm to room temperature and stirred for 1 hour. The solvent is removed and the residue is purified by mass directed preperative HPLC to afford the title material; ¹H NMR (600 MHz, CDCl₃) δ 7.54 (m, 2H), 6.68 (dd, *J* = 8.5, 8.4 Hz, 1H), 4.92 (sept., *J* = 6.3 Hz, 1H), 4.17 (m, 2H), 4.00 (m, 1H), 3.92 (m, 1H), 3.80-3.70 (m, 3H), 3.01 (s, 3H), 2.95 (m, 3H), 2.48 (m, 2H), 2.00 (m, 2H), 1.78 (m, 2H), 1.24 (d, *J* = 6.2 Hz, 6H); ESIMS *m*/*z* for (M+H)⁺ C₂₂H₃₀FN₄O₅S calcd.: 481.6 found: 481.2.

### Example 5

### (S)-3-(1-(5-ethylpyrimidin-2-yl)piperidin-4-yl)-5-(1-(2-fluoro-4-(methylsulfonyl)phenyl)pyrrolidin-3-yl)-1,2,4-oxadiazole

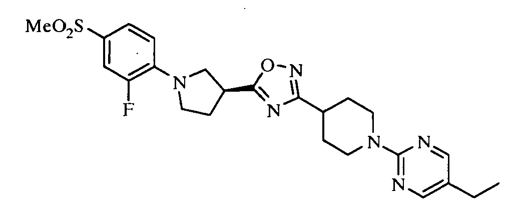

A mixture of intermediate 4a (42 mg, 1.1 mmol), diisopropylethylamine (45 mg, 0.35 mmol) and 2-chloro-5-ethylpyrimidine (30.4 mg, 0.21 mmol) is heated to 80 °C for 5 minutes. The reaction is then treated with CuI (0.7 mg, 0.04 mmol) and the heating is mainitained overnight. The reaction is then purified by preperative mass-directed HPLC to afford the title material;

By repeating the procedures in examples 3-5 or the coupling conditions in example 1, using appropriate starting materials, the following compounds of Formula I, as identified in Table 1, are obtained.

**Table 1**

| **Example #** | **Structure** | **NMR and/or ESMS** |
|---|---|---|
| 6 | 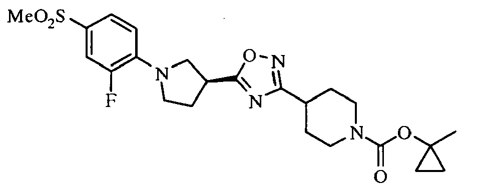 | ¹H NMR (400 MHz, CDCl₃) δ 7.53 (m, 2H), 6.68 (dd, *J* = 8.6, 8.4 Hz, 1H), 4.01 (m, 1H), 3.91 (m, 1H), 3.81-3.68 (m, 3H), 3.02 (s, 3H), 2.92 (m, 3H), 2.46 (m, 2H), 1.99 (m, 2H), 1.75 (m, 2H), 1.60(s, 3H), 1.24 (m, 2H), 0.86 (m, 2H), 0.63 (m, 2H); ESIMS *m*/*z* for (M+H)⁺ C₂₃H₃₀FN₄O₅S calcd.: 493.6 found: 493.1. |
| 7 | 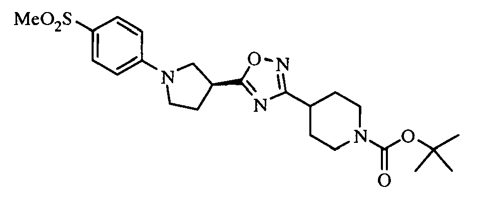 | ¹H NMR (400 MHz, CDCl₃) δ 7.76 (d, *J* = 8.9 Hz, 2H), 6.61 (d, *J* = 9.0 Hz, 2H), 4.12 (m, 2H), 3.85 (m, 2H), 3.74 (m, 1H), 3.64-3.51 (2H), 3.01 (s, 3H), 2.94 (m, 2H), 2.53 (m, 2H), 1.99 (m, 2H), 1.74 (m, 2H), 1.46 (s, 9H); ESIMS *m*/*z* for (M-BOC+H)⁺ C₂₃H₃₃N₄O₅S calcd.: 376.5 found: 377.2. |
| 8 | 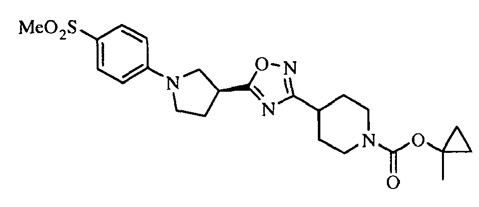 | ¹H NMR (400 MHz, CDCl₃) δ 7.76 (d, *J* = 8.9 Hz, 2H), 6.61 (d, *J* = 9.0 Hz, 2H), 3.85 (m, 2H), 3.74 (m, 1H), 3.62-3.52 (m, 2H), 3.18 (m. 2H), 3.01 (s. 3H), 2.94 (m, 3H), 2.52 (m, 2H), 1.99 (m. 2H), 1.74 (m. 2H), 1.54 (s, 3H). 0.87 (dd, *J* = 6.8. 6.8 Hz. 2H). 0.63 (dd. *J* = 6.8. 5.4 Hz. 2H); ESIMS *m*/*z* for (M+H)⁺ C₂₃H₃₁N₄O₅S calcd.: 475.6 found: 475.2. |
| 9 | 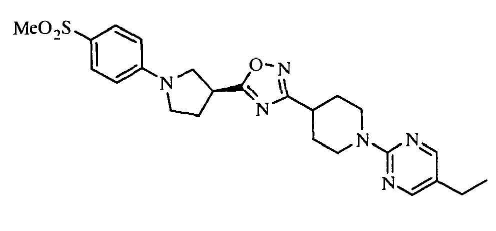 | ¹H NMR (400 MHz. CDCl₃) δ 8.42 (s. 2H), 7.76 (d, *J* = 8.9 Hz, 2H), 6.62 (d, *J* = 9.0 Hz. 2H), 4.57 (m. 2H), 3.86 (m, 2H), 3.75 (m. 2H), 3.62-3.45 (m, 4H). 3.21 (m, 1H). 3.01 (s. 3H). 2.60 (q. *J* = 7.6 Hz. 2H). 2.52 (m, 2H), 2.22 (m. 2H), 1.98 (m. 2H). 1.27 (t, *J* = 7.6 Hz, 3H); ESIMS *m*/*z* for (M+H)⁺ C₂₄H₃₁N₆O₃S calcd.: 483.6 found: 483.3. |

### Example 10

### (S)-isopropyl 4-(5-(1-(2-fluoro-4-(methylsulfonyl)phenyl)pyrrolidin-3-yl)-1,3,4-oxadiazol-2-yl)piperidine-1-carboxylate

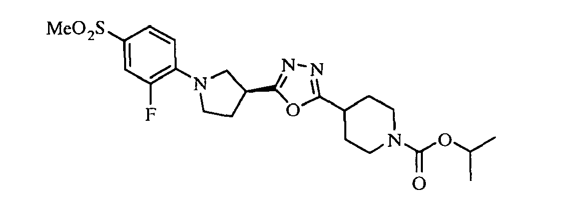

Intermediate 10a: (S)-isopropyl 4-(2-(1-(tert-butoxycarbonyl)pyrrolidine-3-carbonyl)hydrazinecarbonyl)piperidine-1-carboxylate

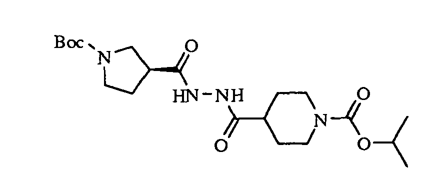

A cold (water/ice bath) solution of (S)-1-(tert-butoxycarbonyl)pyrrolidine-3-carboxylic acid (90.6 mg, 0.42 mmol) in DMF (2.3 mL) is treated with HOBt (77.4 mg, 0.57 mmol), EDCl (79.9 mg, 0.42 mmol). After 30 minutes of stirring, and isopropyl 4-(hydrazinecarbonyl)piperidine-1-carboxylate (99.4 mg, 0.43 mmol) and 4-methylmorpholine (64 mg, 0.54 mmol) are added to the reaction mixure and the reaction is stirred overnight at room temperature. The reaction is diluted with ethyl acetate, extracted with 5% aqueous NaHSO₄, saturated aqueous NaHCO₃ and brine, dried over MgSO₄, filtered and evaporated to afford the title material which was used crude for the next step. ESIMS *m*/*z* for (M+H)⁺ C₂₀H₃₄N₄O₆ calcd.: 426.3 found: 326.3 (M+H-Boc).

Intermediate 10b: (S)-isopropyl 4-(5-(1-(tert-butoxycarbonyl)pyrrolidin-3-yl)-1,3,4-oxadiazol-2-yl)piperidine-1carboxylate

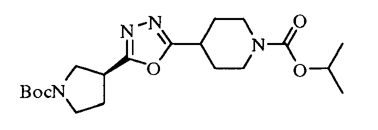

A solution of intermediate 10a (127 mg, 0.30 mmol) and TsCl (113.5 mg, 0.60 mmol) in pyridine (1.5 mL) is heated to reflux for 72 hours. The solvent is removed and the residue is purified by mass directed preperative HPLC to afford the title material. ESIMS *m*/*z* for (M+H)⁺ C₂₀H₃₂N₄O₅ calcd.: 408.2 found: 308.2 (M+H-Boc).

A sample of intermediate 10b (36.1 mg, 0.088 mmol) is treated with a 4M solution of HCl in dioxane and stirred for 1.5 hours. The solvent is then removed and the residue is treated with 1,2-difluoro-4-(methylsulfonyl)benzene (17 mg, 0.88 mmol), K₂CO₃ (36.6 mg, 0.27 mmol) and DMF (1 mL) and heated to 100 °C overnight. The reaction is poured into water and extracted with ethyl acetate twice. The combined organics are dreid over MgSO₄, evaporated and purified by preperative mass directed HPLC to afford (S)-isopropyl 4-(5-(1-(2-ftuoro-4-(methylsulfonyl)phenyl)pyrrolidin-3-yl)-1,3,4-oxadiazol-2-yl)piperidine-1-carboxylate. ¹H NMR (400 MHz, CDCl₃) δ 7.54 (m, 2H), 6.68 (dd, *J* = 8.5, 8.4 Hz, 1H), 4.93 (sept., *J* = 6.2 Hz, 1H). 4.16 (d, *J* = 10.6 Hz, 2H), 3.96 (m, 1H), 3.89 (m, 1H), 3.72 (m, 3H), 3.09 (m, 1H), 3.02 (s, 3H), 2.98 (m, 2H), 2.47 (m, 2H), 2.07 (m, 2H), 1.82 (m, 2H), 1.25 (d, *J* = 6.2 Hz, 6H); ESIMS *m*/*z* for (M+H)⁺ C₂₂H₃₀FN₄O₅S calcd.: 481.6 found: 481.2.

### Example 11

### (S)-isopropyl 4-(4-(1-(2-fluoro-4-(methylsulfonyl)phenyl)pyrrolidin-3-yl)oxazol-2-yl)piperidine-1-carboxylate

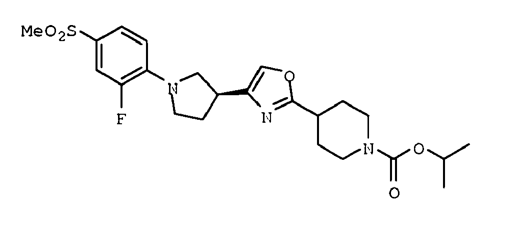

Intermediate 11a: (S)-1-(2-fluoro-4-(methylsulfonyl)phenyl)pyrrolidine-3-carboxylic acid

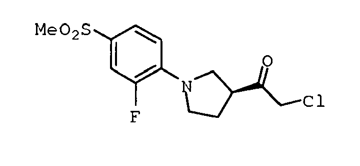

A sample of intermediate 3a (350 mg, 1.22 mmol) is treated with dioxane (3 mL) followed by oxalyl chloride (464 mg, 3.7 mmol) and a drop of DMF. The reaction is stirred for 1 hour and the solvent is removed. The residue is treated with dioxane (3 mL) and trimethylsilyl diazomethane (1.5 mL of a 2 M solution, 3 mmol) and stirred for 3 hours. The reaction is then treated with a 4 M solution of HCl in dioxane (2 mL). Once the bubbling subsides, the solvent is removed and the residue is purified on silica gel using a linear gradient of 0 to 100% ethyl acetate in hexane to afford the title material as a solid; LC/MS calcd. for [M+H]⁺C₁₃H₁₅ClFNO₃S: 319.0, found: 319.1.

A sample of intermediate 10a (50 mg, 0.16 mmol) and isopropyl 4-carbamoylpiperidine-1-carboxylate (100 mg, 0.47 mmol) are heated together with a heat gun until the reaction turns brown (∼1.5 minutes). The reaction is then passed through a plug of silica gel using ethyl acetate as solvent and purified on a mass-triggered HPLC to afford the title material; ¹H NMR (400 MHz, CDCl₃) δ 7.54 (m, 2H), 7.41 (m, 1H), 6.67 (dd, J = 8.5, 8.5 Hz, 1H), 4.93 (m, 1H), 4.16 (m, 2H), 3.87 (m, 1H), 3.67 (m, 3H), 3.47 (m, 1H), 3.04 (s, 3H), 2.97 (m, 2H), 2.46 (m, 1H), 2.05 (m, 1H), 2.21 (m, 1H), 2.03 (m, 2H), 1.80 (m, 2H), 1.27 (d, J = 6.4 Hz, 6H); LC/MS calcd. for [M+H]⁺ C₂₃H₃₀FN₃O₅S: 480.2. found: 480.2.

### Example 12

### (S)-isopropyl 4-(4-(1-(2-fluoro-4-(methylsulfonyl)phenyl)pyrrolidin-3-yl)thiazol-2-yl)piperidine-1-carboxylate

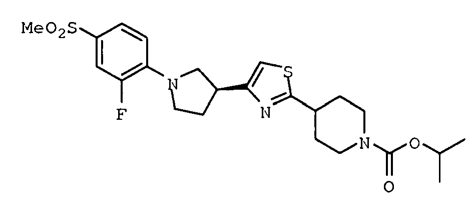

A sample of intermediate 11a (10 mg, 0.031 mmol) is treated with isopropyl 4-carbamothioylpiperidine-1-carboxylate (37 mg, 0.162 mmol) and ethanol (1.5 mL) and heated to 160°C for 5 minutes. The reaction was then purified on the mass triggered HPLC to afford 1.8 mg (12% yield) of the title material: LC/MS calcd. for [M+H]⁺C₂₃H₃₀FN₃O₄S₂: 496.2, found: 496.2.

### Example 13

### 4-{2-[1-(4-Methanesulfonyl-phenyl)-pyrrolidin-3-yl]-S-oxazol-4-yl}-piperidine-1-carboxylic acid isopropyl ester

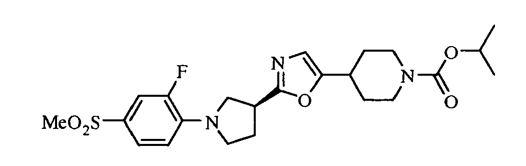

Intermediate 13a: isopropyl 4-(2-chloroacetyl)piperidine-1-carboxylate

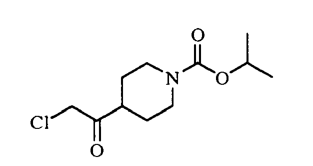

By following the same procedure as intermediate 11a except using 1-(isopropoxycarbonyl)piperidine-4-carboxylic acid as the carboxylic acid, the title material is obtained. LC/MS calcd. for [M+H]⁺C₁₁H₁₈ClNO: 248.1, found: 248.0.

Intermediate 13b: (S)-1-(2-fluoro-4-(methylsulfonyl)phenyl)pyrrolidine-3-carboxamide

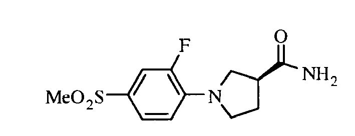

A solution of intermediate 2c (492.9 mg, 1.97 mmol) in DMSO (3 mL) is treated with K₂CO₃ (0.20 g, 0.14 mmol) and immersed in a room temperature water bath. The reaction is then cautiously treated with a 30% aqueous solution of H₂O₂ (500 µL, 4.4 mmol). The reaction is allowed to stir for 30 minutes and poured into water. The resulting solid is collected and dried to afford the title material. LC/MS calcd. for [M+H]⁺ C₁₂H₁₅FN₂O₃S: 287.1, found: 287.1.

By following the same procedure as example 11 eacept using intermediate 13a and intermediate 13b as the coupling partners, the title material is obtained; ¹H NMR (400 MHz, CDCl₃) δ 7.75 (d, *J* = 8.9 Hz, 2H), 7.30 (s, 1H), 6.60 (d, *J* = 9.0 Hz, 2H), 4.92 (sept., J = 6.2 Hz, 1H), 4.20 (bs, 2H), 3.74 (m, 3H), 3.57 (m, 1H), 3.50 (m, 1H), 3.01 (s, 3H), 2.86 (m, 2H), 2.68 (m, 1H), 2.46 (m, 2H), 1.97 (m, 2H), 1.52 (m, 2H), 1.24 (d, *J* = 6.2 Hz, 6H); ESIMS *m*/*z* for (M+H)⁺C₂₃H₃₂N₃O₅S calcd.: 462.6 found: 462.2.

### Example 14

### 4-{2-[1-(2-Fluoro-4-methanesulfonyl-phenyl)-pyrrolidin-3-yl]-S-thiazol-4-yl}-piperidine-1-carboxylic acid isopropyl ester

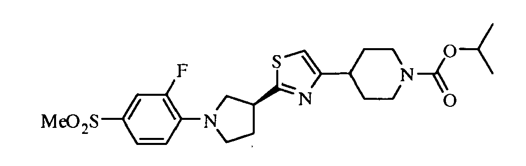

Intermediate 14a: (S)-1-(2-fluoro-4-(methylsulfonyl)phenyl)pyrrolidine-3-carbothioamide

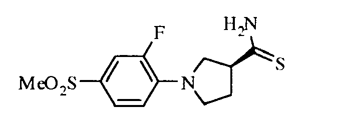

A mixture of intermediate 2c (1.01 g, 3.76 mmol), thioacetamide (720 mg, 9.6 mmol), DMF (1.8 mL) and 4 M HCl in dioxane (3.6 mL) is heated to 100 °C for 2 hours. The reaction is then poured into water and the solid is dissolved in ethyl acetate and extracted with saturated aqueous sodiumhydrogencarbonate and water, dreid over MgSO₄, filtered and evaporated to afford the title material. ESIMS *m*/*z* for (M+H)+C₁₂H₁₅FN₂O₂S₂ calcd.: 303.1 found: 303.2

A mixture of intermediate 13a (55.7 mg, 0.23 mmol) and intermediate 14a (68.0 mg, 2.3 mmol) in ethanol (2 mL) is heated to 80 °C overnight. The reaction is concentrated and purified by mass directed preperative HPLC to afford the title material. ¹H NMR (400 MHz, CDCl₃) δ 7.56 (m, 2H), 6.95 (s, 1H), 6.71 (dd, *J* = 8.5, 8.4 Hz, 1H), 4.93 (sept., *J =* 6.3 Hz, 1H), 4.28 (d, *J =* 10.9 Hz, 1H), 4.17 (m, 1H), 4.00 (m, 1H), 3.80 (m, 2H), 3.67 (m, 1H), 3.07 (m, 1H), 3.03 (s, 3H), 2.90 (m, 2H), 2.60 (m, 1H), 2.29 (m, 1H), 2.06 (d, *J* = 12.6 Hz, 2H), 1.60 (m, 2H), 1.26 (d, *J* = 6.2 Hz, 6H); ESIMS m/z for (M+H)⁺ C₂₃H₃₁FN₃O₄S₂ calcd.: 496.6 found: 496.2.

### Example 15

### (S)-isopropyl 4-(2-(1-(2-fluoro-4-(methylsulfonyl)phenyl)pyrrolidin-3-yl)-2H-tetrazol-5-yl)piperidine-1-carboxylate

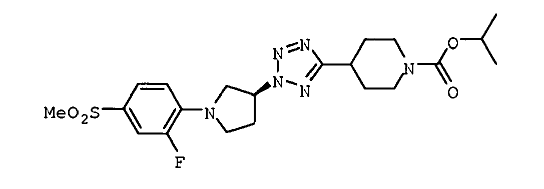

A sample of intermediate 2b (50 mg. 0.15 mmol) is treated with 4-(2H-tetrazol-5-yl)pyridine (26 mg, 0.18 mmol), Cs₂CO₃ (72 mg. 0.22 mmol) and NMP (0.8 mL) heated to 160 °C for 5 minutes in a microwave reactor. The reaction is then diluted with ethyl acetate and extracted once with water. The organics are then extracted with I M HCl twice and discarded. The acidic aqueous washings are made basic with 50% aqueous NaOH, extracted twice with ethyl acetate and discarded. The combined organics are dried over MgSO₄ and evaporated. The residue is treated with 1 mL of AcOH containing TFA (25 mg, 0.22 mmol), MeOH (1 mL) and PtO₂ (20 mg). The reaction is hydrogenated over 1 atmosphere of hydrogen for 72 hours, filtered through celite and evaporated. The residue is treated with ethyl acetate, extracted with 1 M NaOH, dried over MgSO₄, filtered and evaporated. The residue is treated with DCM (2 mL), triethylamine (0.2 mL, xs) and isopropyl chloroformate (0.25 mL of a 1 M solution in toluene). The reaction is allowed to stir for 30 minutes, diluted with ethyl acetate and extracted with 1 M HCl. The organics are then dried over MgSO_{4,} filtered, evaporated and purified on silica gel using a linear gradient of 0-100% ethyl acetate in hexane to afford the title material; ¹H NMR (400 MHz, CDCl₃) δ 7.54 (m, 2H), 6.70 (dd, J = 8.5, 8.5 Hz, 1H), 5.48 (m, 1H), 4.92 (m, 1H), 4.16 (m, 4H), 3.86 (m, 1H), 3.73 (m, 1H), 3.11 (m, 1H), 3.02 (s, 3H), 2.96 (m, 2H), 2.79 (m, 1H), 2.62 (m, 1H), 2.05 (m, 2H), 1.78 (m, 2H), 1.24 (d, J = 6.2 Hz, 6H); LC/MS calcd. for [M+H]⁺ C₂₁H₂₉FN₆O₄S: 481.2, found: 481.2.

### Example 16

### Isopropyl 4-(5-((4-(methylsulfonyl)phenoxy)methyl)-1,2,4-oxadiazol-3-yl)piperidine-1-carboxylate

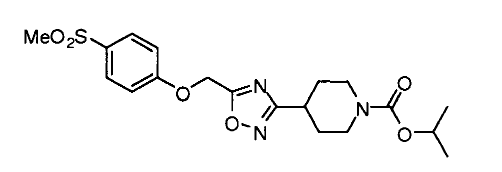

Intermediate 16a: Methyl 2-(4-(methylsulfonyl)phenoxy)acetate

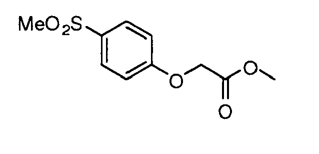

To a solution of 4-methylsulphonylphenol (600 mg, 3.5 mmol) in MeCN (8 mL) are added Cs₂CO₃ (1.7 g, 5.2 mmol) and methylbromoacetate (0.4 mL, 4.3 mmol). The resulting mixture is heated to 60°C for 4 hours. The mixture is then filtered through celite and washed with MeCN. Concentration of the filtrate *in vacuo* affords 851 mg (quant.) of the title material as a white solid. The compound is used in the next step without further purification; ¹H-NMR (600 MHz, CDCl₃) δ = 7.90 (d, *J* = 9.0 Hz, 2H), 7.04 (d, *J* = 9.0 Hz, 2H), 4.74 (s, 2H), 3.84 (s, 3H), 3.05 (s, 3H): MS calcd. for [M+H]⁺ C₁₀H₁₃O₅S: 245.0, found 245.0.

The compounds shown in Table 2 below are synthesized by analogous methods from the appropriate ester derivative:

**Table 2**

| Intermediate | Structure | NMR and/or ESMS |
|---|---|---|
| 17a | 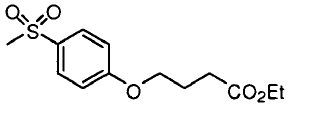 | ¹H-NMR (400 MHz, CD₃CN) δ = 7.85 (d, *J* = 8.4 Hz, 2H), 7.10 (d, *J* = 8.4 Hz, 2H), 4.15-4.09 (m, 4H), 3.03 (s, 3H), 2.49 (t, *J* = 7.2 Hz, 2H), 2.08 (t, *J* = 7.2 Hz, 2H), 1.23 (t. *J* = 7.2 Hz. 3H); MS calcd. for [M+H]⁺ C₁₃H₁₉O₅S: 287.1, found: 287.1. |
| 18a | 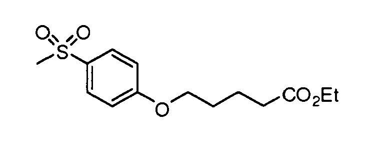 | ¹H-NMR (400 MHz, CD₃CN) δ = 7.85 (d, *J* = 9.2 Hz, 2H), 7.10 (d, *J* = 9.2 Hz, 2H), 4.13-4.07 (m. 4H), 3.04 (s, 3H), 2.38 (t. *J* = 7.6 Hz, 2H), 1.87-1.72 (m, 4H), 1.23 (t, *J* = 7.2 Hz, 3H); MS calcd. for [M+H]⁺ C₁₄H₂₁O₅S: 301.1. found: 301.1. |
| 19a | 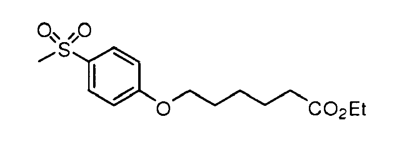 | ¹H-NMR (400 MHz. CD₃CN) δ 7.85 (d. *J* = 8.8 Hz. 2H). 7.10 (d. *J* = 8.8 Hz. 2H). 4.12-4.07 (m. 4H). 3.03 (s. 3H), 233 (t. *J* = 7.6 Hz. 2H). 7.89-1.78 (m. 2H). 1.71-1.58 (m, 2H). 1.54-1.46 (m. 2H). 1.23 (t, *J* = 7.2 Hz. 3H): MS calcd. for [M+H]⁺ C₁₅H₂₃O₅S: 315.1, found: 315.1. |

Intermediate 16b: (Z)-Isopropyl 4-(N'-hydroxycarbamimidoyl)piperidine-1-carboxylate

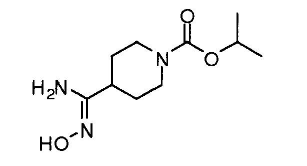

4-Cyanopiperidine (1.36 g, 12.3 mmol) is dissolved in EtOAc and treated with NEt₃ (2.3 mL, 16 mmol). The resulting suspension is cooled to 0°C and isopropyl chloroformate (1M in toluene, 15 mL, 15 mmol) is added dropwise. After completion of the addition, the mixture is stirred overnight at room temperature. The mixture is then filtered over celite and washed with EtOAc. Concentration of the filtrate affords the title compound as a white solid; ¹H-NMR (400 MHz, CDCl₃) δ = 6.87 (br s, 1H), 4.93 (sept., *J* = 6.4 Hz, 1H), 4.51 (s, 2H), 4.23 (br s, 2H), 2.79 (t, *J* = 12.4 Hz, 2H), 2.33-2.26 (m, 1H), 1.85 (d, *J* = 12.4 Hz, 2H), 1.62-1.53 (m, 2H), 1.26 (d, *J* = 6.0 Hz, 6H); MS calcd. for [M+H]⁺ C₁₀H₂₀N₃O3: 230.1, found 230.1.

A sample of intermediate **16a** (73.3 mg, 0.30 mmol) and amide oxime **16b** (68.8 mg, 0.30 mmol) are suspended in 3:1 toluene/DMA (2 mL) and heated to 110°C for 3 days. The reaction is then filtered through a syringe filter using DMA as solvent and purified on a mass-triggered HPLC to afford of title material; ¹H-NMR (400 MHz, CD₃CN) δ = 7.91 (d, *J* = 8.8 Hz, 2H), 7.22 (d, *J* = 8.8 Hz, 2H), 5.45 (s, 2H), 4.86 (sept., *J* = 6.4 Hz, 1H), 4.12-4.05 (m, 2H), 3.06 (s, 3H), 3.1-2.92 (m, 4H), 2.25-2.00 (m, 2H), 1.72-1.60 (m, 2H), 1.23 (d, *J* = 6.4 Hz, 6H); MS calcd. for [M+H]⁺ C₁₉H₂₆N₃O₆S: 423.1, found: 423.1.

### Example 17

### Isopropyl 4-(5-(3-(4-(methylsulfonyl)phenoxy)propyl)-1,2,4-oxadiazol-3-yl)piperidine-1-carboxylate

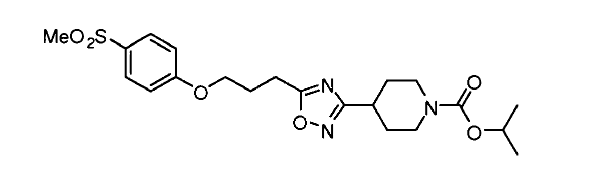

A sample of intermediate **16b** (68.8 mg, 0.30 mmol) is dissolved in THF (3 mL) and NaH (60% dispersion in mineral oil, 12 mg, 0.3 mmol) is added at room temperature. The resulting suspension is heated to 60°C for 2.5 hours. The reaction mixture is then cooled to room temperature and activated 4Å molecular sieves (powder, 250 mg) are added followed by a solution of the ester **17a** in THF (0.7 mL). Stirring of the reaction mixture is continued at 60°C overnight. The reaction is then filtered through a syringe filter using MeCN as solvent and purified on a mass-triegered HPLC to afford the title material; ¹H-NMR (400 MHz, CD₃CN) δ = 7.85 (d, *J* = 8.8 Hz, 2H), 7.07 (d, *J* = 8.8 Hz, 2H), 4.85 (sept., *J* = 6.4 Hz, 1H), 4.19 (t, *J* = 6.0 Hz, 2H), 4.11-4.02 (m, 2H), 3.07 (t, *J =* 7.2 Hz, 2H), 3.04 (s, 3H), 3.01-2.93 (m, 2H), 2.32-2.25 (m, 2H), 1.63 (dq, *J* = 11.6, 4.0 Hz, 2H), 1.23 (d, *J* = 6.4 Hz, 6H); MS calcd. for [M+H]⁺C₂₁H₃₀N₃O₆S: 452.2, found: 452.0.

The compounds shown in Table 3 below are synthesized by analogous methods from intermediate **16b** and the appropriate esters:

**Table 3**

| **Example #** | **Structure** | **NMR and/or ESMS** |
|---|---|---|
| 18 | 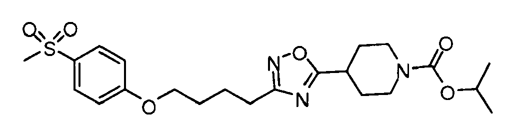 | ¹H-NMR (400 MHz. CD₃CN) δ = 7.85(d,*J*=8.8Hz,2H),7.10(d,*J*= 8.8 Hz, 2H), 4.85 (sept., *J* = 6.0 Hz. 1H), 4.13 (t. *J* = 6.0 Hz. 2H), 4.11-4.03 (m, 2H), 3.04 (s, 3H). 3.00-2.93 (m, 2H), 1.94-1.86 (m, 2H), 1.63 (dq. *J* = 11.6, 4.4 Hz. 2H). 1.23 (t, *J* = 6.0 Hz. 3H); MS calcd. for [M+H]⁺ C₂₂H₃₂N₃O₆S: 466.2. found: 466.0. |
| 19 | 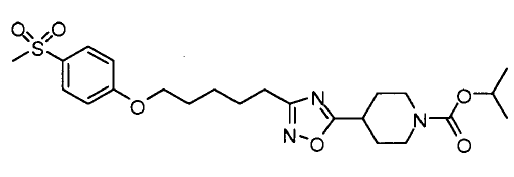 | ¹H-NMR (400 MHz. CD₃CN) δ = 7.85 (d. *J* = 8.8 Hz. 2H). 7.09 (d. *J* = 8.8 Hz. 2H), 4.85 (sept., *J.* = 6.4 Hz. 1H), 4.11-4.05 (m, 4H), 3.04 (s, 3H), 3.01-2.93 (m, 2H). 2.91 (t. *J*= 7.6 Hz, 2H), 1.90-1.80 (m, 2H), 1.69-1.51 (m. 4H), 1.23 (t. *J* = 6.4 Hz, 3H); MS calcd. for [M+H]⁺ C₂₃H₃₄N₃O₆S: 480.2, found: 480.0. |

### Biological Assays

**Generation of Stable Cell Line**

Flp-In-CHO cells (Invitrogen, Cat.# R758-07) are maintained in Ham's F12 medium supplemented with 10% fetal bovine serum, 1% antibiotic mixture and 2mM L-glutamine. The cells are transfected with a DNA mixture containing human GPR119 in pcDNA5/FRT vector and the pOG44 vector (1:9) using Fugene6 (Roche), according to the manufacturer's instruction. After 48 hours, the medium is changed to medium supplemented with 400µg/ml hygromycin B to initiate the selection of stably transfected cells.

**Cyclic AMP Assay in Stable Cell Line**

To test the activity of compounds of the invention, Flp-In-CHO-hGPR119 cells are harvested and resuspended in DMEM plus 3% lipid-depleted fetal bovine serum. Forth µl of cells are plated in 384 well plates at a density of 15,000 cells/well. IBMX (3-isobutyl-1-methyl-xanthine) is added to the cells to a final concentration of ImM, followed by the addition of 500nl of the compound to be tested. The cells are incubated at 37°C for 30 minutes. Equal volume (20µl) of the HTRF reagents, anti-cAMP-Cryptate and cAMP-XL665, are added to the cells. The plates are incubated at room temperature for 1 hour and read on a HTRF reader according to the manufacturer's instruction.

Compounds of Formula I, in free form or in pharmaceutically acceptable salt form, produced a concentration-dependent increase in intracellular cAMP level. Compound of the invention show an EC₅₀ of between 1x10⁻⁵ and 1x 10⁻¹⁰M, preferably less than 500nM, more preferably less than 100nM.

It is understood that the specific examples and embodiments described herein are for illustrative purposes only and that various modifications or changes in light thereof will be suggested to persons skilled in the art within the scope of the appended claims.

## Claims

1. A compound of Formula I: 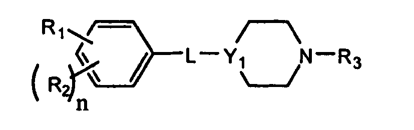 in which:
n is selected from 0,1,2 and 3;
L is selected from: -Y₂X₃-; -OX₁X₃-; -OX₁OX₃-; -OX₁C(O)X₂X₃₋; - OX₁C(O)OX₂X₃-; and -OC(O)NR₄X₁X₃-; wherein Y₂ is a 5 to 8 member heterocyclic containing 1 to 3 heteroatoms selected from O, N and S; X₁ and X₂ are independently selected from a bond, C₁₋₆alkylene, C₂₋₆alkenylene, C₃₋₈cycloalkyl and C₁₋₅heteroarylene; R₄ is selected from hydrogen and C₁₋₆alkyl; and X₃ is selected from 1,2,4-oxadiazole, 1,3,4-oxadiazole, oxazole, thiazole and tetrazole;
R₁ is selected from C₁₋₄alkyl, halo-substituted-C₁₋₄alkyl, C₆₋₁₀aryl, -X₄S(O)₀₋₂ R₅ₐ, -X₄C(O)OR₅ₐ, -X₄OR₅ₐ, - X₄C(O)R₅ₐ, - X₄C(O)NR₅ₐR_{5b}, -X₄NR_{5c}S(O)0-2R₅ₐ, - X₄NR_{5c}C(O)OR₅ₐ, - X₄NR_{5c}C(O)R₅ₐ, and - X₄NR₅,C(O)NR₅ₐR_{5b}; wherein R₅ₐ and R_{5b} are independently selected from hydrogen, C₁₋₆alkyl, C₁₋₆alkoxy, halo-substituted-C₁₋₆alkyl, halo-substituted-C₁₋₆alkoxy and C₁₋₁₀heteroaryl, wherein X₄ is selected from a bond, C₁₋₃alkylene and C₃₋₆cycloalkylene; R_{5c} is selected from hydrogen, C₁₋₆alkyl, C₃₋₆cycloalkyl, C₃₋₈ heterocycloalkyl, C₆₋₁₀aryl and C₁₋₁₀heteroaryl; wherein any alkyl, cycloalkyl, aryl or heteroaryl of R_{5c} can be optionally substituted with 1 to 3 radicals independently selected from halo, C₁₋₆alkyl, C₁₋₆alkoxy, halo-substituted-C₁₋₆alkyl and halo-substituted-C₁₋₆alkoxy;
R₂ is selected from halo, cyano, C₁₋₈alkyl, C₁₋₈alkoxy, halo-substituted-C₁₋₈ alkyl, halo-substituted-C₁₋₈alkoxy and nitro;
R₃ is selected from C₁₋₁₀heteroaryl, -C(O)OR₆ₐ, -C(O)R₆ₐ, -S(O)₀₋₂R₆ₐ, - C(O)R₇ and -C(O)X₅NR₆ₐC(O)OR_{6b}; wherein X₅ is selected from a bond and C₁₋₆alkylene; R₆ₐ and R_{6b} are independently selected from hydrogen and C₁₋₆alkyl; R₇ is selected from C₃₋₈ cycloalkyl and C₆₋₁₀aryl; wherein said heteroaryl of R₃ is optionally substituted with 1 to 3 radicals independently selected from halo, C₁₋₆alkyl, C₁₋₆alkoxy, halo-substituted-C₁₋₆alkyl, halo-substituted-C₁₋₆alkoxy, C₃₋₈heterocycloalkyl and C₁₋₁₀heteroaryl; and
Y₁ is selected from CR₈ and N; wherein R₈ is selected from hydrogen and C₁₋₆ alkyl; or a pharmaceuticaly acceptable salt thereof

2. The compound of claim 1 in which:
n is selected from 0, 1 and 2;
L is selected from -Y₂X₃- and -OX₁X₃-; wherein Y₂ is a 5 to 8 member heterocyclic containing 1 to 3 heteroatoms selected from O, N and S; X₁ is selected from a bond, C₁₋₆alkylene and C₂₋₆alkenylene; and X₃ is selected from 1,2,4-oxadiazole, 1,3,4-oxadiazole, oxazole, thiazole and tetrazole;
R₁ is selected from halo, cyano, C₁₋₄alkyl, halo-substituted-C₁₋₄alkyl, C₆₋₁₀aryl, -S(O)₀₋₂R₅ₐ, -C(O)OR₅ₐ, -C(O)R₅ₐ, and -C(O)NR₅ₐR_{5b}; wherein R₅ₐ and R_{5b} are independently selected from hydrogen, C₁₋₄alkyl and halo-substituted-C₁₋₄alkyl;
R₂ is selected from halo and nitro;
R₃ is selected from C₁₋₁₀heteroaryl, -C(O)OR₆ₐ, -C(O)R₆ₐ, -S(O)₀₋₂R₆ₐ, - C(O)R₇ and O-C(O)X₅NR₆ₐC(O)OR_{6b}; wherein X₅ is selected from a bond and C₁₋₄alkylene; R₆ₐ and R_{6b} are independently selected from hydrogen and C₁₋₆alkyl; R₇ is selected from C₃₋₈ cycloalkyl and C₆₋₁₀aryl; wherein said heteroaryl of R₃ is optionally substituted with up to three C₁₋₆alkyl radicals; and
Y₁ is selected from CR₈ and N; wherein R₈ is selected from hydrogen and C₁₋₄ alkyl.

3. The compound of claim 2 in which L is selected from -Y₂X₃- and - OX₁X₃-; wherein Y₂ is pyrrolidine; X₁ is selected from methylene, propylene, butylene and pentylene; and X₃ is selected from 1,2,4-oxadiazole, 1,3,4-oxadiazole, oxazole, thiazole and tetrazole.

4. The compound of claim 3 in which R₁ is selected from halo, cyano, trifluoromethyl, methyl-sulfonyl, methoxy-carbonyl, phenyl, methyl-carbonyl, amino-carbonyl and trifluoromethyl-sulfonyl.

5. The compound of claim 4 in which R₃ is selected from t-butoxy-carbonyl, isopropoxy-carbonyl, t-butyl-carbonyl, t-butyl-methyl-carbonyl, t-butoxy-carbonylaminomethyl-carbonyl, phenyl-carbonyl, cyclohexyl-carbonyl, t-butyl-sulfinyl, pyridinyl, isopropyl-sulfonyl and ethyl-pyrimidinyl.

6. The compound of claim 1 selected from: tert-butyl 4-(3-(1-(4-(methylsulfonyl)phenyl)pyrrolidin-3-yl)-1,2,4-oxadiazol-5-yl)piperidine-1-carboxylate; (S)-isopropyl 4-(3-(1-(2-fluoro-4-(methylsulfonyl)phenyl)pyrrolidin-3-yl)-1,2,4-oxadiazol-5-yl)piperidine-1-carboxylate; (S)-tert-butyl 4-(5-(1-(2-fluoro-4-(methylsulfonyl)phenyl)pyrrolidin-3-yl)-1,2,4-oxadiazol-3-yl)piperidine-1-carboxylate; (S)-isopropyl 4-(5-(1-(2-fluoro-4-(methylsulfonyl)phenyl)pyrrolidin-3-yl)-1,2,4-oxadiazol-3-yl)piperidine-1-carboxylate; (S)-3-(1-(5-ethylpyrimidin-2-yl)piperidin-4-yl)-5-(1-(2-fluoro-4-(methylsulfonyl)phenyl)pyrrolidin-3-yl)-1,2,4-oxadiazole; (S)-1-methylcyclopropyl 4-(5-(1-(2-fluoro-4-(methylsulfonyl)phenyl)pyrrolidin-3-yl)-1 1,2,4-oxadiazol-3-yl)piperidine-1-carboxylate; (S)-tert-butyl 4-(5-(1-(4-(methylsulfonyl)phenyl)pyrrolidin-3-yl)-1,2,4-oxadiazol-3-yl)piperidine-1-carboxylate; (S)-1-methylcyclopropyl 4-(5-(1-(4-(methylsulfonyl)phenyl)pyrrolidin-3-yl)-1,2,4-oxadiazol-3-yl)piperidine-1-carboxylate; (S)-3-(1-(5-ethylpyrimidin-2-yl)piperidin-4-yl)-5-(1-(4-(methylsulfonyl)phenyl)pyrrolidin-3-yl)-1,2,4-oxadiazole; (S)-isopropyl 4-(5-(1-(2-fluoro-4-(methylsulfonyl)phenyl)pyrrolidin-3-yl)-1,3,4-oxadiazol-2-yl)piperidine-1-carboxylate; (S)-isopropyl 4-(4-(1-(2-fluoro-4-(methylsulfonyl)phenyl)pyrrolidin-3-yl)oxazol-2-yl)piperidine-1-carboxylate; (S)-isopropyl 4-(4-(1-(2-fluoro-4-(methylsulfonyl)phenyl)pyrrolidin-3-yl)thiazol-2-yl)piperidine-1-carboxylate; 4-{2-[1-(4-Methanesulfonyl-phenyl)-pyrrolidin-3-yl]-S-oxazol-4-yl}-piperidine-1-carboxylic acid isopropyl ester; 4-{2-[1-(2-Fluoro-4-methanesulfonyl-phenyl)-pyrrolidin-3-yl]-S-thiazol-4-yl}-piperidine-1-carboxylic acid isopropyl ester; (S)-isopropyl 4-(2-(1-(2-fluoro-4-(methylsulfonyl)phenyl)pyrrolidin-3-yl)-2H-tetrazol-5-yl)piperidine-1-carboxylate; isopropyl 4-(5-((4-(methylsulfonyl)phenoxy)methyl)-1,2,4-oxadiazol-3-yl)piperidine-1-carboxylate; isopropyl 4-(5-(3-(4-(methylsulfonyl)phenoxy)propyl)-1,2,4-oxadiazol-3-yl)piperidine-1-carboxylate; isopropyl 4-(3-(4-(4-(methylsulfonyl)phenoxy)butyl)-1,2,4-oxadiazol-5-yl)piperidine-1-carboxylate; and isopropyl 4-(3-(5-(4-(methylsulfonyl)phenoxy)pentyl)-1,2,4-oxadiazol-5-yl)piperidine-1-carboxylate.

7. A pharmaceutical composition comprising a therapeutically effective amount of a compound of any one of claims 1 to 6 in combination with a pharmaceutically acceptable excipient.

8. A compound of any one of claims 1 to 6 for use in the treatment of a disease or condition selected from obesity, type 1 diabetes, type 2 diabetes mellitus, hyperlipidemia, idiopathic type 1 diabetes, latent autoimmune diabetes in adults, early-onset type 2 diabetes, youth-onset atypical diabetes, maturity onset diabetes of the young, malnutrition-related diabetes and gestational diabetes.

9. A compound of any one of claims 1 to 6 for use in the treatment of a disease or condition selected from coronary heart disease, ischemic stroke, restenosis after angioplasty, peripheral vascular disease, intermittent claudication, myocardial infarction, dyslipidemia, post-prandial lipemia, conditions of impaired glucose tolerance, conditions of impaired fasting plasma glucose, metabolic acidosis, ketosis, arthritis, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertrygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance.

10. An in vitro method for modulating GPR119 activity, comprising administering to a system, an effective amount of the compound of any one of claims 1 to 6 or pharmaceutically acceptable salts or pharmaceutical compositions thereof, thereby modulating said GPR119 activity.

11. The method of claim 10, wherein the compound of any one of claim 1 to 6 directly contacts GPR119.

## Patentansprüche

1. Verbindung der Formel I: 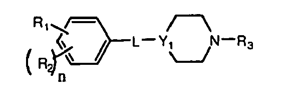 worin:
n aus 0, 1, 2 und 3 ausgewählt ist;
L aus -Y₂Y₃-; -OX₁X₃-; -OX₁OX₃-; -OX₁C(O)X₂X₃-; -OX₁C(O)OX₂X₃-; und -OC(O)NR₄X₁X₃- ausgewählt ist; worin Y₂ ein 5- bis 8-gliedriges Heterocyclyl ist, das 1 bis 3 aus O, N und S ausgewählte Heteroatome enthält; X₁ und X₂ unabhängig voneinander aus einer Bindung, C₁₋₆-Alkylen, C₂₋₆-Alkenylen, C₃₋₈-Cycloalkyl, C₁₋₅-Heteroarylen ausgewählt sind; R₄ aus Wasserstoff und C₁₋₆-Alkyl ausgewählt ist; und X₃ aus 1,2,4-Oxadiazol, 1,3,4-Oxadiazol, Oxazol, Thiazol und Tetrazol ausgewählt ist;
R₁ aus C₁₋₄-Alkyl, halogensubstituiertem C₁₋₄-Alkyl, C₆₋₁₀-Aryl, -X₄S(O)₀₋₂R₅ₐ, -X₄C(O)OR₅ₐ, -X₄OR₅ₐ, -X₄C(O)R₅ₐ, -X₄C(O)NR₅ₐR_{5b}, -X₄NR_{5c}(S(O)₀₋₂R₅ₐ, -X₄NR_{5c}-C(O)OR₅ₐ, -X₄NR_{5c}C(O)R₅ₐ und -X₄NR_{5c}C(O)NR₅ₐR_{5b} ausgewählt ist; worin R₅ₐ und R_{5b} unabhängig voneinander aus Wasserstoff, C₁₋₆-Alkyl, C₁₋₆-Alkoxy, halogensubstituiertem C₁₋₆-Alkyl, halogensubstituiertem C₁₋₆-Alkoxy und C₁₋₁₀-Heteroaryl ausgewählt sind, worin X₄ aus einer Bindung, C₁₋₃-Alkylen und C₃₋₆-Cycloalkylen ausgewählt ist; R_{5c} aus Wasserstoff, C₁₋₆-Alkyl, C₃₋₆-Cycloalkyl, C₃₋₈-Heterocycloalkyl, C₆₋₁₀-Aryl und C₁₋₁₀-Heteroaryl ausgewählt ist; worin jedes Alkyl, Cycloalkyl, Aryl oder Heteroaryl von R_{5c} gegebenenfalls mit 1 bis 3 unabhängig voneinander aus Halogen, C₁₋₆-Alkyl, halogensubstituiertem C₁₋₆-Alkyl und halogensubstituiertem C₁₋₆-Alkoxy ausgewählten Resten substituiert ist;
R₂ aus Halogen, Cyano, C₁₋₈-Alkyl, C₁₋₈-Alkoxy, halogensubstituiertem C₁₋₈-Alkyl, halogensubstituiertem C₁₋₈-Alkoxy und Nitro ausgewählt ist;
R₃ aus C₁₋₁₀-Heteroaryl, -C(O)OR₆ₐ, -C(O)R₆ₐ, -S(O)₀₋₂R₆ₐ, -C(O)R₇ und -C(O)X₅NR₆ₐC(O)OR_{6b} ausgewählt ist; worin X₅ aus einer Bindung und C₁₋₆-Alkylen ausgewählt ist; R₆ₐ und R_{6b} unabhängig voneinander aus Wasserstoff und C₁₋₆-Alkyl ausgewählt sind; R₇ aus C₃₋₈-Cycloalkyl und C₆₋₁₀-Aryl ausgewählt ist; worin das Heteroaryl von R₃ gegebenenfalls mit 1 bis 3 unabhängig voneinander aus Halogen, C₁₋₆-Alkyl, C₁₋₆-Alkoxy, halogensubstituiertem C₁₋₆-Alkyl, halogensubstituiertem C₁₋₆-Alkoxy, C₃₋₈-Heterocycloalkyl und C₁₋₁₀-Heteroaryl ausgewählten Resten substituiert ist; und
Y₁ aus CR₈ und N ausgewählt ist; worin R₈ aus Wasserstoff und C₁₋₆-Alkyl ausgewählt ist;
oder ein pharmazeutisch annehmbares Salz davon.

2. Verbindung nach Anspruch 1, worin:
n aus 0, 1 und 2 ausgewählt ist;
L aus -Y₂Y₃- und -OX₁X₃- ausgewählt ist; worin Y₂ ein 5- bis 8-gliedriges Heterocyclyl ist, das 1 bis 3 aus O, N und S ausgewählte Heteroatome enthält; X₁ aus einer Bindung, C₁₋₆-Alkylen und C₂₋₆-Alkenylen ausgewählt ist; und X₃ aus 1,2,4-Oxadiazol, 1,3,4-Oxadiazol, Oxazol, Thiazol und Tetrazol ausgewählt ist;
R₁ aus Halogen, Cyano, C₁₋₄-Alkyl, halogensubstituiertem C₁₋₄-Alkyl, C₆₋₁₀-Aryl, -S(O)₀₋₂R₅ₐ, -C(O)OR₅ₐ, -C(O)R₅ₐ und -C(O)NR₅ₐR_{5b} ausgewählt ist; worin R₅ₐ und R_{5b} unabhängig voneinander aus Wasserstoff, C₁₋₄-Alkyl und halogensubstituiertem C₁₋₄-Alkyl ausgewählt sind;
R₂ aus Halogen und Nitro ausgewählt ist;
R₃ aus C₁₋₁₀-Heteroaryl, -C(O)OR₆ₐ, -C(O)R₆ₐ, -S(O)₀₋₂R₆a, -C(O)R₇ und -C(O)X₅NR₆ₐC(O)OR_{6b} ausgewählt ist; worin X₅ aus einer Bindung und C₁₋₄-Alkylen ausgewählt ist; R₆ₐ und R_{6b} unabhängig voneinander aus Wasserstoff und C₁₋₆-Alkyl ausgewählt sind; R₇ aus C₃₋₈-Cy_{d}oalkyl und C₆₋₁₀-Aryl ausgewählt ist; worin das Heteroaryl von R₃ gegebenenfalls mit bis zu drei C₁₋₆-Alkylresten substituiert ist; und
Y₁ aus CR₈ und N ausgewählt ist; worin R₈ aus Wasserstoff und C₁₋₄-Alkyl ausgewählt ist.

3. Verbindung nach Anspruch 2, worin L aus -Y₂Y₃- und -OX₁X₃- ausgewählt ist; worin Y₂ Pyrrolidin ist; X₁ aus Methylen, Propylen, Butylen und Pentylen ausgewählt ist; und X₃ aus 1,2,4-Oxadiazol, 1,3,4-Oxadiazol, Oxazol, Thiazol und Tetrazol ausgewählt ist.

4. Verbindung nach Anspruch 3, worin R₁ aus Halogen, Cyano, Trifluormethyl, Methylsulfonyl, Methoxycarbonyl, Phenyl, Methylcarbonyl, Aminocarbonyl und Trifluormethylsulfonyl ausgewählt ist.

5. Verbindung nach Anspruch 4, worin R₃ aus t-Butoxycarbonyl, Isopropoxy-carbonyl, t-Butylcarbonyl, t-Butylmethylcarbonyl, t-Butoxycarbonylaminomethylcarbonyl, Phenylcarbonyl, Cyclohexylcarbonyl, t-Butylsulfinyl, Pyridinyl, Isopropylsulfonyl und Ethylpyrimidinyl ausgewählt ist.

6. Verbindung nach Anspruch 1, ausgewählt aus: tert-Butyl-4-(3-(1-(4-(methylsulfonyl)phenyl)pyrrolidin-3-yl)-1,2,4-oxadiazol-5-yl)piperidin-1-carboxylat; (S)-Isopropyl-4-(3-(1-(2-fluor-4-(methylsulfonyl)phenyl)pyrrolidin-3-yl)-1,2,4-oxadiazol-5-yl)-piperidin-1-carboxylat; (S)-tert-Butyl-4-(5-(1-(2-fluor-4-(methylsulfonyl)phenyl)pyrrolidin-3-yl)-1,2,4-oxadiazol-3-yl)piperidin-1-carboxylat; (S)-Isopropyl-4-(5-(1-(2-fluor-4-(methylsulfonyl)phenyl)pyrrolidin-3-yl)-1,2,4-oxadiazol-3-yl)piperidin-1-carboxylat; (S)-3-(1-(5-Ethylpyrimidin-2-yl)piperidin-4-yl)-5-(1-(2-fluor-4-(methylsulfonyl)phenyl)-pyrrolidin-3-yl)-1,2,4-oxadiazol; (S)-1-Methylcyclopropyl-4-(5-(1-(2-fluor-4-(methylsulfonyl)phenyl)pyrrolidin-3-yl)-1,2,4-oxadiazol-3-yl)piperidin-1-carboxylat; (S)-tert-Butyl-4-(5-(1-(4-(methylsulfonyl)phenyl)pyrrolidin-3-yl)-1,2,4-oxadiazol-3-yl)piperidin-1-carboxylat; (S)-1-Methylcyclopropyl-4-(5-(1-(4-(methylsulfonyl)phenyl)pyrrolidin-3-yl)-1,2,4-oxadiazol-3-yl)piperidin-1-carboxylat; (S)-3-(1-(5-Ethylpyrimidin-2-yl)piperidin-4-yl)-5-(1-(4-(methylsulfonyl)phenyl)pyrrolidin-3-yl)-1,2,4-oxadiazol; (S)-Isopropyl-4-(5-(1-(2-fluor-4-(methylsulfonyl)phenyl)pyrrolidin-3-yl)-1,3,4-oxadiazol-2-yl)piperidin-1-carboxylat; (S)-Isopropyl-4-(4-(1-(2-fluor-4-(methylsulfonyl)phenyl)pyrrolidin-3-yl)oxazol-2-yl)piperidin-1-carboxylat; (S)-Isopropyl-4-(4-(1-(2-fluor-4-(methylsulfonyl)-phenyl)pyrrolidin-3-yl)thiazol-2-yl)piperidin-1-carboxylat; 4-{2-[1-(4-Methansulfonylphenyl)pyrrolidin-3-yl]-S-oxazol-4-yl}piperidin-1-carbonsäureisopropylester; 4-{2-[1-(2-Fluor-4-methansulfonylphenyl)pyrrolidin-3-yl]-S-thiazol-4-yl}piperidin-1-carbonsäureisopropylester; (S)-Isopropyl-4-(2-(1-(2-fluor-4-(methylsulfonyl)phenyl)pyrrolidin-3-yl)-2H-tetrazol-5-yl)piperidin-1-carboxylat; Isopropyl-4-(5-((4-(methylsulfonyl)-phenoxy)methyl)-1,2,4-oxadiazol-3-yl)piperidin-1 -carboxylat; Isopropyl-4-(5-(3-(4-(methylsulfonyl)phenoxy)propyl)-1,2,4-oxadiazol-3-yl)piperidin-1-carboxylat; Isopropyl-4-(3-(4-(4-(methylsulfonyl)phenoxy)butyl)-1,2,4-oxadiazol-5-yl)piperidin-1-carboxylat; und Isopropyl-4-(3-(5-(4-(methylsulfonyl)phenoxy)pentyl)-1,2,4-oxadiazol-5-yl)-piperidin-1-carboxylat.

7. Pharmazeutische Zusammensetzung, umfassend eine therapeutisch wirksame Menge einer Verbindung nach einem der Ansprüche 1 bis 6 in Kombination mit einem pharmazeutisch annehmbaren Exzipienten.

8. Verbindung nach einem der Ansprüche 1 bis 6 zur Verwendung bei der Behandlung einer aus Adipositas, Typ-I-Diabetes, Typ-II-Diabetes mellitus, Hyperlipidämie, idiopathischem Typ-I-Diabetes, latentem Autoimmundiabetes bei Erwachsenen, Early-onset-Typ-II-Diabetes, juvenilem atypischem Diabetes, MODY, unterernährungsbedingtem Diabetes und Schwangerschaftsdiabetes ausgewählten Krankheit oder Störung.

9. Verbindung nach einem der Ansprüche 1 bis 6 zur Verwendung bei der Behandlung einer aus koronarer Herzkrankheit, ischämischem Schlaganfall, Restenose nach Angioplastie, peripherer Gefäßkrankheit, Claudicatio intermittens, Myokardinfarkt, Dyslipidämie, postprandialer Lipämie, Leiden auf Grund von gestörter Glucosetoleranz, Leiden auf Grund von gestörter Nüchtern-Plasma-Glucose, metabolischer Azidose, Ketose, Arthritis, Osteoporose, Hypertonie, kongestiver Herzinsuffizienz, linksventrikulärer Hypertrophie, peripherer Arterienkrankheit, diabetischer Retinopathie, Makuladegeneration, Katarakt, diabetischer Nephropathie, Glomerulosklerose, chronischer Niereninsuffizienz, diabetischer Neuropathie, metabolischem Syndrom, Syndrom X, prämenstrualem Syndrom, koronarer Herzkrankheit, Angina pectoris, Thrombose, Atherosklerose, Myokardinfarkt, transitorischen ischämischen Attacken, Schlaganfall, Gefäßrestenose, Hyperglykämie, Hyperinsulinämie, Hyperlipidämie, Hypertriglyceridämie, Insulinresistenz, gestörtem Glucosemetabolismus, Leiden auf Grund von gestörter Glucosetoleranz, Leiden auf Grund von gestörter Nüchtern-Plasma-Glucose, Adipositas, erektiler Dysfunktion, Haut- und Bindegewebeerkrankungen, Fußulzerationen und Colitis ulcerosa, Endotheldysfunktion und gestörter Gefäßcompliance ausgewählten Krankheit oder Störung.

10. In-vitro-Verfahren zum Modulieren der Aktivität von GPR119, umfassend das Verabreichen einer wirksamen Menge der Verbindung nach einem der Ansprüche 1 bis 6 oder pharmazeutisch annehmbarer Salze oder pharmazeutischer Zusammensetzungen davon an ein System und das Modulieren der GPR119-Aktivität **dadurch**.

11. Verfahren nach Anspruch 10, worin die Verbindung nach einem der Ansprüche 1 bis 6 GPR119 direkt kontaktiert.

## Revendications

1. Composé de formule I: 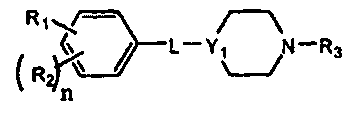 dans laquelle:
n est choisi parmi 0, 1, 2 et 3;
L est choisi parmi: -Y₂-X₃-; -OX₁X₃-; -OX₁X₃-; -OX₁C(O)X₂X₃-; -OX₁C(O)OX₂X₃- et -OC(O)NR₄X₁X₃-; où Y₂ est un groupe hétérocyclique à 5 à 8 chaînons contenant 1 à 3 hétéroatomes choisis parmi O, N et S; X₁ et X₂ sont indépendamment choisis parmi une liaison, un groupe alkylène en C₁ à 6, alcénylène en C_{2 à} 6, cycloalkyle en C_{3 à 8} et hétéroarylène en C_{1 à 5}; R₄ est choisi parmi un atome d'hydrogène et un groupe alkyle en C_{1 à 6}; et X₃ est choisi parmi un groupe 1,2,4-oxadiazole, 1,3,4-oxadiazole, oxazole, thiazole et tétrazole;
R₁ est choisi parmi un groupe alkyle en C_{1 à 4}, alkyle en C_{1 à} 4 halogéno-substitué, aryle en C_{6 à 10}, -X₄S (O) _{0 à 2}R₅ₐ, -X₄C(O) OR₅ₐ, -X₄OR₅ₐ, -X₄C (O) R₅ₐ, -X₄C (O)NR₅ₐR_{5b}, -X₄NR_{5c}S(O)_{0 à 2}R₅ₐ, -X₄NR_{5c}C(O)OR₅ₐ, -X₄NR_{5c}C(O)R₅ₐ et -X₄NR_{5c}C (O) NR₅ₐR_{5b}; où R₅ₐ et R_{5b} sont indépendamment choisis parmi un atome d'hydrogène, un groupe alkyle en C_{1 à 6}, alcoxy en C_{1 à 6}, alkyle en C_{1 à 6} halogéno-substitué, alcoxy en C_{1 à 6} halogéno-substitué et hétéroaryle en C_{1 à 10}, où X₄ est choisi parmi une liaison, un groupe alkylène en C₁ à ₃ et cycloalkylène en C_{3 à 6}; R_{5c} est choisi parmi un atome d'hydrogène, un groupe alkyle en C_{1 à 6}, cycloalkyle en C_{3 à 6}, hétérocycloalkyle en C_{3 à 8,} aryle en C_{6 à 10} et hétéroaryle en C_{1 à 10}; où tout groupe alkyle, cycloalkyle, aryle ou hétéroaryle de R_{5c} peut être facultativement substitué par 1 à 3 radicaux indépendamment choisis parmi un groupe halogéno, alkyle en C_{1 à 6,} alcoxy en C_{1 à 6}, alkyle en C_{1 à 6} halogéno-substitué et alcoxy en C_{1 à 6} halogéno-substitué;
R₂ est choisi parmi un groupe halogéno, cyano, alkyle en C₁ à ₈, alcoxy en C_{1 à 8}, alkyle en C_{1 à 8} halogéno-substitué, alcoxy en C_{1 à 8} halogéno-substitué et nitro;
R₃ est choisi parmi un groupe hétéroaryle en C_{1 à 10}, -C (O)OR₆ₐ, -C(O)R₆ₐ, -S (O)_{0 à 2} R₆ₐ, -C(O)R₇ et -C(O)X₅NR₆ₐC (O) OR_{6b;} où X₅ est choisi parmi une liaison et un groupe alkylène en C_{1 à 6}; R₆ₐ et R_{6b} sont indépendamment choisis parmi un atome d'hydrogène et un groupe alkyle en C_{1 à 6}; R₇ est choisi parmi un groupe cycloalkyle en C_{3 à 8} est aryle en C_{6 à 10}; où ledit groupe hétéroaryle de R₃ est facultativement substitué par 1 à 3 radicaux indépendamment choisis parmi un groupe halogéno, alkyle en C_{1 à 6}, alcoxy en C_{1 à 6}, alkyle en C_{1 à 6} halogéno-substitué, alcoxy en C_{1 à 6} halogéno-substitué, hétérocycloalkyle en C_{3 à 8} et hétéroaryle en C_{1 à 10}; et
Y₁ est choisi parmi CR₈ et N; où R₈ est choisi parmi un atome d'hydrogène et un groupe alkyle en C_{1 à 6}; ou sel pharmaceutiquement acceptable de celui-ci

2. Composé selon la revendication 1, dans lequel:
n est choisi parmi 0, 1 et 2;
L est choisi parmi -Y₂X₃- et -OX₁X₃-; où Y₂ est un groupe hétérocyclique à 5 à 8 chaînons contenant 1 à 3 hétéroatomes choisis parmi O, N et S; X₁ est choisi parmi une liaison, un groupe alkylène en C_{1 à 6} et alcénylène en C_{2 à 6}; et X₃ est choisi parmi un groupe 1,2,4-oxadiazole, 1,3,4-oxadiazole, oxazole, thiazole et tétrazole;
R₁ est choisi parmi un groupe halogéno, cyano, alkyle en C_{1 à 4}, alkyle en C_{1 à 4} halogéno-substitué, aryle en C_{6 à 10}, -S (O) _{0 à 2}R₅ₐ, -C(O) OR₅ₐ, -C(O)R₅ₐ et -C(O)NR₅ₐR_{5b}; où R₅ₐ et R_{5b} sont indépendamment choisis parmi un atome d'hydrogène, un groupe alkyle en C_{1 à 4} et alkyle en C_{1 à 4} halogéno-substitué;
R₂ est choisi parmi un groupe halogéno et nitro;
R₃ est choisi parmi un groupe hétéroaryle en C_{1 à 10}, -C (O) OR₆ₐ, -C(O)R₆ₐ, -S(O)_{0 à 2}R₆ₐ, -C(O)R₇ et -C (O) X₅NR₆ₐC (O) OR_{6b;} où X₅ est choisi parmi une liaison et un groupe alkylène en C_{1 à 4}; R₆ₐ et R_{6b} sont indépendamment choisis parmi un atome d'hydrogène et un groupe alkyle en C_{1 à 6}; R₇ est choisi parmi un groupe cycloalkyle en C_{3 à 8} et aryle en C_{6 à 10}; où ledit groupe hétéroaryle de R₃ est facultativement substitué par jusqu'à trois radicaux alkyle en C_{1 à 6}; et
Y₁ est choisi parmi CR₈ et N; où R₈ est choisi parmi un atome d'hydrogène et un groupe alkyle en C_{1 à 4}.

3. Composé selon la revendication 2, dans lequel L est choisi parmi -Y₂X₃- et -OX₁X₃- ; où Y₂ est un groupe pyrrolidine; X₁ est choisi parmi un groupe méthylène, propylène, butylène et pentylène; et X₃ est choisi parmi un groupe 1,2,4-oxadiazole, 1,3,4-oxadiazole, oxazole, thiazole et tétrazole.

4. Composé selon la revendication 3 dans lequel R₁ est choisi parmi un groupe halogéno, cyano, trifluorométhyle, méthyl-sulfonyle, méthoxy-carbonyle, phényle, méthyl-carbonyle, amino-carbonyle et trifluorométhyl-sulfonyle.

5. Composé selon la revendication 4, dans lequel R₃ est choisi parmi un groupe t-butoxy-carbonyle, isopropoxy-carbonyle, t-butyl-carbonyle, t-butyl-méthyl-carbonyle, t-butoxy-carbonyle, aminométhyl-carbonyle, phényl-carbonyle, cyclohexyl-carbonyle, t-butyl-sulfinyle, pyridinyle, isopropyl-sulfonyle et éthyl-pyrimidinyle.

6. Composé selon la revendication 1 choisi parmi le 4-(3-(1-(4-méthylsulfonyl)phényl)pyrrolidin-3-yl)-1,2,4-oxadiazol-5-yl)pipéridine-1-carboxylate de tert-butyle; le 4-(3-(1-(2-fluoro-4-(méthylsulfonyl)phényl)pyrrolidin-3-yl)-1,2,4-oxadiazol-5-yl)pipéridine-1-carboxylate de (S)-isopropyle; le 4-(5-(1-(2-fluoro-4-(méthyl-sulfonyl)phényl)pyrrolidin-3-yl)-1,2,4-oxadiazol-3-yl)pipéridine-1-carboxylate de (S)-tert-butyle; le 4-(5-(1-(2-fluoro-(4-(méthylsulfonyl)phényl)pyrrolidin-3-yl)-1,2,4-oxadiazol-3-yl)pipéridine-1-carboxylate de (S)-isopropyle; le (S)-3-(1-(5-éthylpyrimidin-2-yl)pipéridin-4-yl)-5-(1-(2-fluoro-4-(méthylsulfonyl)phényl)pyrrolidin-3-yl)-1,2,4-oxadiazole; le 4-(5-(1-(2-fluoro-4-(méthyl-sulfonyl)phényl)pyrrolidin-3-yl)-1,2,4-oxadiazol-3-yl)pipéridine-1-carboxylate de (S)-1-méthylcyclopropyle; le 4-(5-(1-(4-(méthylsulfonyl)phényl)pyrrolidin-3-yl)-1,2,4-oxadiazol-3-yl)pipéridine-1-carboxylate de (S)-tert-butyle; le 4-(5-(1-(4-(méthylsulfonyl)phényl)pyrrolidin-3-yl)-1,2,4-oxadiazol-3-yl)pipéridine-1-carboxylate de (S)-1-méthylcyclopropyle; le (S)-3-(1-(5-éthylpyrimidin-2-yl)pipéridin-4-yl)-5-(1-(4-(méthylsulfonyl)phényl)pyrrolidin-3-yl)-1,2,4-oxadiazole;
le 4-(5-(1-(2-fluoro-4-(méthylsulfonyl)phényl)pyrrolidin-3-yl)-1,3,4-oxadiazol-2-yl)pipéridine-1-carboxylate de (S)-isopropyle; le 4-(4-(1-(2-fluoro-4-(méthyl-sulfonyl)phényl)pyrrolidin-3-yl)oxazol-2-yl)pipéridine-1-carboxylate de (S)-isopropyle; le 4-(4-(1-(2-fluoro-4-(méthylsulfonyl)phényl)pyrrolidin-3-yl)thiazol-2-yl)pipéridine-1-carboxylate de (S)-isopropyle; l'ester d'isopropyle d'acide 4-{2-[1-(4-méthanesulfonyl-phényl)-pyrrolidin-3-yl]-*S*-oxazol-4-yl}-pipéridine-1-carboxylique; l'ester d'isopropyle d'acide 4-{2-[1-(2-fluoro-4-méthanesulfonyl-phényl)-pyrrolidin-3-yl]-S-thiazol-4-yl}-pipéridine-1-carboxylique; le 4-(2-(1-(2-fluoro-4-(méthylsulfonyl)phényl)pyrrolidin-3-yl)-2H-tétrazol-5-yl)pipéridine-1-carboxylate de (S)-isopropyle; le 4-(5-((4-(méthylsulfonyl)phénoxy)méthyl)-1,2,4-oxadiazol-3-yl)pipéridine-1-carboxylate d'isopropyle; le 4-(5-(3-(4-(méthylsulfonyl)phénoxy)propyl)-1,2,4-oxadiazol-3-yl)pipéridine-1-carboxylate d'isopropyle; le 4-(3-(4-(4-(méthylsulfonyl)phénoxy)butyl)-1,2,4-oxadiazol-5-yl)pipéridine-1-carboxylate d'isopropyle et le 4-(3-(5-(4-(méthylsulfonyl)phénoxy)pentyl)-1,2,4-oxadiazol-5-yl)pipéridine-1-carboxylate d'isopropyle.

7. Composition pharmaceutique comprenant une quantité thérapeutiquement efficace d'un composé selon l'une quelconque des revendications 1 à 6 en combinaison avec un excipient pharmaceutiquement acceptable.

8. Composé selon l'une quelconque des revendications 1 à 6, à utiliser dans le traitement d'une maladie ou d'une affection choisie parmi l'obésité, le diabète de type 1, le diabète sucré de type 2, l'hyperlipidémie, le diabète de type 1 idiopathique, le diabète latent auto-immun de l'adulte, le diabète de type 2 précoce, le diabète atypique juvénile, le diabète de type 2 de la jeune personne, le diabète associé à la malnutrition et le diabète gestationnel.

9. Composé selon l'une quelconque des revendications 1 à 6 à utiliser dans le traitement d'une maladie ou d'une affection choisie parmi une maladie cardiaque coronarienne, un accident ischémique, une resténose après angioplastie, une maladie vasculaire périphérique, une claudication intermittente, l'infarctus du myocarde, la dyslipidémie, la lipidémie post-prandiale, les affections d'intolérance au glucose, les affections d'intolérance au glucose du plasma, l'acidose métabolique, la cétose, l'arthrite, l'ostéoporose, l'hypertension, l'insuffisance cardiaque congestive, l'hypertrophie ventriculaire gauche, une maladie artérielle périphérique, la rétinopathie diabétique, la dégénérescence maculaire, la cataracte, la néphropathie diabétique, la glomérulosclérose, l'insuffisance rénale chronique, une neuropathie diabétique, le syndrome métabolique, le syndrome X, le syndrome prémenstruel, une maladie cardiaque coronarienne, l'angine de poitrine, la thrombose, l'athérosclérose, l'infarctus du myocarde, les accidents ischémiques transitoires, l'accident vasculaire cérébral, la resténose vasculaire, l'hyperglycémie, l'hyperinsulinémie, l'hyperlipidémie, l'hypertriglycéridémie, la résistance à l'insuline, l'intolérance au glucose métabolique, les affections d'intolérance au glucose, les affections d'intolérance au glucose du plasma, l'obésité, les troubles de l'érection, les troubles cutanés et du tissu conjonctif, les ulcères du pied et la rectocolite hémorragique, un dysfonctionnement endothélial et la non-compliance vasculaire.

10. Procédé *in vitro* pour moduler l'activité de GPR119, comprenant l'administration à un système, d'une quantité efficace du composé selon l'une quelconque des revendications 1 à 6 ou de sels pharmaceutiquement acceptables ou de compositions pharmaceutiques de celui-ci, modulant ainsi ladite activité de GPR119.

11. Procédé selon la revendication 10, dans lequel le composé selon l'une quelconque des revendications 1 à 6 entre directement en contact avec GPR119.
